# EUROPEAN PATENT APPLICATION

(11) **EP 4 840 880 A1**
(43) Date of publication of application: **18.11.2026**
(21) Application number: 25884444.8
(22) Date of filing: 15.09.2025
(51) Int. Cl.: C07K 14/54, C07K 14/715, A61P 35/00, A61K 39/395, A61K 38/00, A61K 38/20, A61K 45/06

(54) **DIMER COMPOUND OF NOVEL PROTEIN COMPLEX, PREPARATION METHOD THEREFOR, AND PHARMACEUTICAL USE THEREOF**

(30) Priority: 30.10.2024 CN 202411535077
(71) Applicant: Qianhangjiang Pharmaceutical Co., Ltd., Jiaxing, Zhejiang 314000 (CN)
(72) Inventor: JIANG, Julian Xuliang, Jiaxing, Zhejiang 314000 (CN); QIAN, Jenny Naifeng, Jiaxing, Zhejiang 314000 (CN); LIU, Yongliang, Jiaxing, Zhejiang 314000 (CN)
(74) Representative: Houghton, Mark Phillip
(86) International application number: PCT/CN2025/121245
(87) International publication number: WO 2026/091928

(57) **Abstract**

The present invention relates to a heterodimeric protein and to methods for its preparation and pharmaceutical uses thereof. The heterodimeric protein comprises an IL-2/IL-15 chimeric polypeptide and an IL-15Rα-Fc fusion polypeptide, wherein the IL-2/IL-15 chimeric polypeptide comprises an N-terminal polypeptide segment derived from IL-2 fused to a full-length IL-15 polypeptide or a variant thereof, and wherein the IL-15Rα-Fc fusion polypeptide comprises the Sushi domain of IL-15Rα fused to the CH2-CH3 domains of an Fc region. Two heterodimeric units associate via Fc-mediated dimerization to form a homodimeric complex. The heterodimeric protein is capable of activating and promoting proliferation of immune cells and is suitable for use in the treatment of cancer.

## Description

This application claims priority to Chinese patent application No. 2024115350779, filed October 30, 2024, the entire contents of which are incorporated herein by reference.

### Technical Field

The present invention relates to the field of biotechnology, in particular to heterodimeric proteins each comprising an IL-2/IL-15 chimeric polypeptide chain and an IL-15Rα-Fc fusion polypeptide chain, wherein the heterodimeric proteins are capable of forming homodimers by Fc-mediated dimerization, methods for preparing such proteins, and uses thereof in therapy.

### Background of the Invention

Interleukins (ILs) are a family of cytokines primarily expressed and secreted by leukocytes. They play important roles in the proliferation, differentiation, and immunomodulation of immune cells, including T cells and natural killer (NK) cells. Many interleukins contribute to antitumor activity through diverse mechanisms. Interleukin-15 (IL-15) is a member of the IL-2 cytokine family. Its heterotrimeric receptor, IL-15R, shares a common gamma chain (γc) with receptors for other members of the family, including IL-2, IL-4, IL-7, IL-9, and IL-21. IL-15 enhances both innate and adaptive immune responses by stimulating CD8⁺ and CD4⁺ T cells as well as NK cells. Unlike IL-2, IL-15 does not activate regulatory T (Treg) cells and does not induce activation-induced cell death (AICD) in effector T cells or NK cells, both of which are associated with downregulation of immune responses.

Multiple studies have investigated the anticancer efficacy of interleukins-such as IL-2, IL-7, IL-12, IL-15, IL-18, IL-21, and IL-23-used alone or in the form of interleukin complexes comprising a receptor subunit with a ligand-binding domain (e.g., Anktiva) across various cancer types. Among these interleukins, interleukin-2 (IL-2) is one of the most extensively studied and has been approved for the treatment of metastatic melanoma and metastatic renal cell carcinoma. IL-2 therapy has been reported to induce tumor regression and, in some cases, durable remission. In vitro, IL-2 promotes the activation and proliferation of anti-tumor immune cells, including tumor-infiltrating T cells, chimeric antigen receptor (CAR)-T cells and NK cells. However, IL-2-expanded immune cells tend to exhibit a more differentiated phenotype, which may negatively affect their long-term persistence and survival.

Owing to its IL-2-like antitumor activity, along with the advantages of not activating regulatory T cells, avoiding AICD, and a generally favorable toxicity profile, IL-15 is considered a promising cytokine for cancer immunotherapy. In addition to its use as a monotherapy, IL-15 has been incorporated into adoptive cell therapies, particularly those involving chimeric antigen receptor (CAR) engineering. For example, IL-15 may be used for ex vivo expansion of CAR-T cells or may be genetically incorporated into CAR constructs, thereby enabling co-expression of IL-15 by CAR-T cells. Such co-expression is believed to allow IL-15 to function in an autocrine manner to promote CAR-T cell proliferation and persistence. In addition, in the field of cancer vaccines, IL-15 may be co-administered with tumor antigens to stimulate the proliferation and activation of NK cells, CD8⁺ cytotoxic T cells, CD4⁺ helper T cells, and memory T cells, thereby promoting durable antitumor immune responses.

However, native IL-15 presents challenges for drug development, including low expression levels, a short half-life, and limited developability. Accordingly, there remains a need for IL-15-based therapeutics to have improved expression, extended half-life and enhanced biological activity. IL-15 functions naturally via a trans-presentation mechanism, wherein IL-15 first binds to the α subunit of its receptor (IL-15Rα) on one cell. The resulting IL-15/IL-15Rα complex then interacts with a βγ receptor dimer (IL-2/IL-15Rβ/γc) on a neighboring cell, thereby initiating downstream intracellular signaling. Based on this mechanism, complexes comprising IL-15 and the Sushi domain of soluble IL-15Rα have been developed as receptor agonists. These complexes have been reported to exhibit increased expression and to be more effective than IL-15 alone in stimulating the proliferation of memory CD8⁺ T cells and NK cells, as well as in maintaining the viability of memory CD8⁺ T cells. Antitumor studies of such IL-15/Sushi domain complexes have demonstrated enhanced potency.

To prolong the half-life of a therapeutic protein, a common strategy is fusion with an Fc domain. This approach increases the molecular size of the protein, thereby reducing renal clearance, and enables binding to the neonatal Fc receptor (FcRn), which recycles the protein and extends its circulating half-life. Examples include IL-15Rα-linker-IL-15 (RLI), N-803 (ALT-803), and NKTR-255. Among these, N-803 has been approved for use in the treatment of Bacillus Calmette-Guérin (BCG)-unresponsive non-muscle invasive bladder cancer (NMIBC) with carcinoma in situ (CIS), with or without papillary tumors.

Another approach for extending half-life is to increase glycosylation. A representative example is the long-acting erythropoiesis-stimulating agent darbepoetin alfa, which comprises additional glycosylation sites relative to native erythropoietin (EPO), resulting in an extended in vivo half-life and a reduced dosing frequency.

Although IL-15 and IL-2 are closely related cytokines with analogous structures and functions, their expression profiles differ: IL-2 is expressed at relatively high levels, whereas IL-15 expression is tightly regulated at the transcriptional, translational, and intracellular transport levels, which is associated with structural divergence in that IL-15 lacks an N-terminal segment present in IL-2 that contains an O-linked glycosylation site; accordingly, the present invention provides a chimeric protein comprising IL-2 and IL-15 sequences, wherein the chimeric protein is capable of forming a complex with an IL-15Rα-Fc (CH2-CH3) fusion protein to form a heterodimer, and wherein two such heterodimers associate via Fc-mediated dimerization to form a homodimeric complex, the resulting structure comprising two identical chimeric IL-2/IL-15 polypeptide chains and two identical IL-15Rα-Fc fusion polypeptide chains. Compared with N-803, the proteins of the present invention exhibit higher expression, increased glycosylation, an extended in vivo half-life, enhanced biological activity, and improved developability.

### Detailed Description of the Invention

The present invention relates to heterodimeric proteins comprising an IL-2/IL-15 chimeric polypeptide chain and an IL-15Rα-Fc fusion polypeptide chain, wherein two heterodimeric units are capable of associating via Fc-mediated dimerization to form a homodimeric complex. Fc-mediated homodimerization is well established in the art and is therefore not described in detail herein. The present invention further relates to methods for preparing the proteins and uses thereof for treating cancers.

In one aspect, the IL-2/IL-15 chimeric polypeptide chain comprises an amino acid sequence in which an N-terminal IL-2 segment is fused to a full-length IL-15 sequence or a variant thereof. And the other chain, the IL-15Rα-Fc fusion polypeptide chain, comprises the Sushi domain of IL-15Rα linked to the CH2-CH3 domains of an antibody heavy chain constant region, which is a construct known in the art.

The proteins of the present invention include QHJ-02V03, QHJ-02V05, QHJ-02V13, QHJ-02V15, and QHJ-02M02.

In particular embodiments:
QHJ-02V03 comprises an IL-2/IL-15 chimeric polypeptide chain comprising the amino acid sequence of SEQ ID NO: 7 and an IL-15Rα-Fc fusion polypeptide chain comprising the amino acid sequence of SEQ ID NO: 6; alternatively, QHJ-02V03 comprises an IL-2/IL-15 chimeric polypeptide chain comprising an amino acid sequence having at least 80% sequence identity to SEQ ID NO: 7 and an IL-15Rα-Fc fusion polypeptide chain comprising an amino acid sequence having at least 80% sequence identity to SEQ ID NO: 6.
QHJ-02V05 comprises an IL-2/IL-15 chimeric polypeptide chain comprising the amino acid sequence of SEQ ID NO: 8 and an IL-15Rα-Fc fusion polypeptide chain comprising the amino acid sequence of SEQ ID NO: 6; alternatively, QHJ-02V05 comprises an IL-2/IL-15 chimeric polypeptide chain comprising an amino acid sequence having at least 80% sequence identity to SEQ ID NO: 8 and an IL-15Rα-Fc fusion polypeptide chain comprising an amino acid sequence having at least 80% sequence identity to SEQ ID NO: 6.
QHJ-02V13 comprises an IL-2/IL-15 chimeric polypeptide chain comprising the amino acid sequence of SEQ ID NO: 9 and an IL-15Rα-Fc fusion polypeptide chain comprising the amino acid sequence of SEQ ID NO: 6; alternatively, QHJ-02V13 comprises an IL-2/IL-15 chimeric polypeptide chain comprising an amino acid sequence having at least 80% sequence identity to SEQ ID NO: 9 and an IL-15Rα-Fc fusion polypeptide chain comprising an amino acid sequence having at least 80% sequence identity to SEQ ID NO: 6.
QHJ-02V15 comprises an IL-2/IL-15 chimeric polypeptide chain comprising the amino acid sequence of SEQ ID NO: 9 and an IL-15Rα-Fc fusion polypeptide chain comprising the amino acid sequence of SEQ ID NO: 10; alternatively, QHJ-02V15 comprises an IL-2/IL-15 chimeric polypeptide chain comprising an amino acid sequence having at least 80% sequence identity to SEQ ID NO: 9 and an IL-15Rα-Fc fusion polypeptide chain comprising an amino acid sequence having at least 80% sequence identity to SEQ ID NO: 10.
QHJ-02M02 comprises an IL-2/IL-15 chimeric polypeptide chain comprising the amino acid sequence of SEQ ID NO: 9 and an IL-15Rα-Fc fusion polypeptide chain comprising the amino acid sequence of SEQ ID NO: 11; alternatively, QHJ-02M02 comprises an IL-2/IL-15 chimeric polypeptide chain comprising an amino acid sequence having at least 80% sequence identity to SEQ ID NO: 9 and an IL-15Rα-Fc fusion polypeptide chain comprising an amino acid sequence having at least 80% sequence identity to SEQ ID NO: 11.
QHJ-02V13 and QHJ-02M02 comprise the same IL-2/IL-15 chimeric polypeptide chain (SEQ ID NO: 9), and their IL-15Rα-Fc fusion polypeptide chains share 97.4% sequence identity. Similarly, QHJ-02V15 and QHJ-02M02 comprise the same IL-2/IL-15 chimeric polypeptide chain (SEQ ID NO: 9), and their IL-15Rα-Fc fusion polypeptide chains share 95.7% sequence identity.

Specifically, the IL-2/IL-15 chimeric polypeptide comprises amino acid sequences having sequence identity of at least 80%, 81%, 82%, 83%, 84%, 85%, 86%, 87%, 88%, 89%, 90%, 91%, 92%, 93%, 94%, 95%, 96%, 97%, 98%, or 99% to SEQ ID NO: 7.

Specifically, the IL-15Rα-Fc fusion polypeptide comprises amino acid sequences having sequence identity of at least 80%, 81%, 82%, 83%, 84%, 85%, 86%, 87%, 88%, 89%, 90%, 91%, 92%, 93%, 94%, 95%, 96%, 97%, 98%, 99% to SEQ ID NO: 6.

Specifically, the IL-2/IL-15 chimeric polypeptide comprises amino acid sequences having sequence identity of at least 80%, 81%, 82%, 83%, 84%, 85%, 86%, 87%, 88%, 89%, 90%, 91%, 92%, 93%, 94%, 95%, 96%, 97%, 98%, or 99% to SEQ ID NO: 8.

Specifically, the IL-2/IL-15 chimeric polypeptide comprises amino acid sequences having sequence identity of at least 80%, 81%, 82%, 83%, 84%, 85%, 86%, 87%, 88%, 89%, 90%, 91%, 92%, 93%, 94%, 95%, 96%, 97%, 98%, or 99% to SEQ ID NO: 9.

Specifically, the IL-15Rα-Fc fusion polypeptide comprises amino acid sequences having sequence identity of at least 80%, 81%, 82%, 83%, 84%, 85%, 86%, 87%, 88%, 89%, 90%, 91%, 92%, 93%, 94%, 95%, 96%, 97%, 98%, 99% to SEQ ID NO: 10.

Specifically, the IL-15Rα-Fc fusion polypeptide comprises amino acid sequences having sequence identity of at least 80%, 81%, 82%, 83%, 84%, 85%, 86%, 87%, 88%, 89%, 90%, 91%, 92%, 93%, 94%, 95%, 96%, 97%, 98%, 99% to SEQ ID NO: 11.

Specifically, the IL-2/IL-15 chimeric polypeptide comprises a partial sequence of human IL-2 having a glycosylation site fused with human IL-15, thereby providing a protein with higher molecular weight, increased glycosylation, and higher expression levels than IL-15 alone.

In some embodiments, the IL-2-derived sequence consists of 5 to 30 amino acids, preferably 6 to 15 amino acids.

In preferred embodiments, the IL-2-derived sequence comprises one or more glycosylation sites. In preferred embodiments, the IL-2-derived sequence has at least 60% sequence identity to APTSSSTKKTQL. More preferably, the IL-2-derived sequence consists of APTSSSTKKTQL, corresponding to the N-terminal 12 amino acids of native human IL-2.
**SEQ ID NO: 7:** 
APTSSSTKKTQLNWVNVISDLKKIEDLIQSMHIDATLYTESDVHPSCKVTAMKCFLLELQ
VISLESGDASIHDTVENLIILANDSLSSNGNVTESGCKECEELEEKNIKEFLQSFVHIVQMFI
NTS.
**SEQ ID NO: 6:** 
ITCPPPMSVEHADIWVKSYSLYSRERYICNSGFKRKAGTSSLTECVLNKATNVAHWTTPS
LKCIREPKSCDKTHTCPPCPAPELLGGPSVFLFPPKPKDTLMISRTPEVTCVVVDVSHEDPE
VKFNWYVDGVEVHNAKTKPREEQYNSTYRVVSVLTVLHQDWLNGKEYKCKVSNKALP
APIEKTISKAKGQPREPQVYTLPPSRDELTKNQVSLTCLVKGFYPSDIAVEWESNGQPENN
YKTTPPVLDSDGSFFLYSKLTVDKSRWQQGNVFSCSVMHEALHNHYTQKSLSLSPGK.
**SEQ ID NO: 8:** 
APTSSSTKKTQLNWVNVISDLKKIEDLIQSMHIDATLYTESDVHPSCKVTAMKCFLLELQ
VISLESGDASIHDTVENLIILANDSLSSNGNVTESGCKECEELEEKNIKEFLQSFVHIVQMFI
NTSDYKDDDDK.
**SEQ ID NO: 9:** 
APTSSSTKKTQLNWVNVISDLKKIEDLIQSMHIDATLYTESDVHPSCKVTAMKCFLLELQ
VISLESGDASIHDTVENLIILANNSLSSNGNVTESGCKECEELEEKNIKEFLQSFVHIVQMFI
NTS.
**SEQ ID NO: 10:** 
ITCPPPMSVEHADIWVKSYSLYSRERYICNSGFKRKAGTSSLTECVLNKATNVAHWTTPS
LKCIREPKSCDKTHTCPPCPAPEFEGGPSVFLFPPKPKDTLMISRTPEVTCVVVDVSHEDPE
VKFNWYVDGVEVHNAKTKPREEQYNSTYRVVSVLTVLHQDWLNGKEYKCKVSNKALP
ASIEKTISKAKGQPREPQVYTLPPSREEMTKNQVSLTCLVKGFYPSDIAVEWESNGQPEN
NYKTTPPVLDSDGSFFLYSKLTVDKSRWQQGNVFSCSVMHEALHNHYTQKSLSLSPGK.
**SEQ ID NO: 11:**

ITCPPPMSVEHADIWVKSYSLYSRERYICNSGFKRKAGTSSLTECVLNKATNVAHWTTPS
LKCIRDPALVHQRPKSCDKTHTCPPCPAPELLGGPSVFLFPPKPKDTLMISRTPEVTCVVV
DVSHEDPEVKFNWYVDGVEVHNAKTKPREEQYNSTYRVVSVLTVLHQDWLNGKEYKC

KVSNKALPAPIEKTISKAKGQPREPQVYTLPPSRDELTKNQVSLTCLVKGFYPSDIAVEWE
SNGQPENNYKTTPPVLDSDGSFFLYSKLTVDKSRWQQGNVFSCSVMHEALHNHYTQKSL
SLSPGK.

In another aspect, the present invention relates to a method for preparing the proteins, comprising the steps of:
1). constructing expression plasmids, each comprising a nucleic acid sequence encoding either the IL-2/IL-15 chimeric polypeptide or the IL-15Rα-Fc fusion polypeptide;
2). culturing host cells and co-transfecting the cells with the expression plasmids using a transfection reagent; and
3). adding a transfection enhancer to the culture, maintaining the culture under conditions suitable for protein expression, and subsequently harvesting the protein by centrifugation and collecting a protein-containing supernatant.

In some embodiments, in step 2, the host cells are cultured at 37°C, 80% relative humidity, and 8% CO₂ in a culture medium that may comprise penicillin and streptomycin. The host cells may be selected from Expi293F cells, CHO cells, and HEK293 cells, preferably Expi293F cells. The culture medium may be selected from BalanceCD 293 medium, MRS medium, M17 medium, PDA medium, LB medium, TB medium, SOB medium, MEM medium, DMEM medium, and RPMI-1640 medium, preferably BalanceCD 293 medium.

In some embodiments, in step 2, the ratio of the plasmid encoding the IL-2/IL-15 chimeric polypeptide to the plasmid encoding the IL-15Rα-Fc fusion polypeptide is from 1:1 to 5:1, such as 1:1 or 5:1, preferably 1:1. The total amount of plasmids may be 100 µg per 100 mL of cell culture. The transfection time in step 2 may be from 16 h to 20 h, preferably about 20 h.

In some embodiments, the transfection enhancer in step 3 comprises glucose solution and valproic acid (VPA) solution, wherein the volume ratio of the glucose solution (vGlucose) to the VPA solution (vGlucose/vVPA ) is from 5:1 to 8:1, preferably about 7:1. The glucose solution may have a concentration of about 400 g/L, and the VPA solution may have a concentration of about 66.7 g/L. The amount of transfection enhancer added may be 20 mL/L of cell culture. The centrifugation in step 3 may be carried out at 4°C at 2,000-8,000 rpm for 5 to 10 minutes.

In another aspect, the present invention provides nucleic acid molecules encoding proteins including QHJ-02V03, QHJ-02V05, QHJ-02V13, QHJ-02V15, and QHJ-02M02. The nucleic acid molecules corresponding to the proteins are designated using the same codes as the proteins.

In some embodiments, QHJ-02V03 comprises nucleic acid molecules encoding an IL-2/IL-15 chimeric polypeptide chain and an IL-15Rα-Fc fusion polypeptide chain, wherein the respective ORFs comprise SEQ ID NO: 12 and SEQ ID NO: 13. Alternatively, QHJ-02V03 comprises nucleic acid molecules encoding the same polypeptide chains, wherein the respective ORFs comprise nucleic acid sequences having at least 70% sequence identity to SEQ ID NO: 12 and SEQ ID NO: 13.

QHJ-02V05 comprises nucleic acid molecules encoding an IL-2/IL-15 chimeric polypeptide chain and an IL-15Rα-Fc fusion polypeptide chain, wherein the respective ORFs comprise SEQ ID NO: 14 and SEQ ID NO: 13. Alternatively, QHJ-02V05 comprises nucleic acid molecules encoding the same polypeptide chains, wherein the respective ORFs comprise nucleic acid sequences having at least 70% sequence identity to SEQ ID NO: 14 and SEQ ID NO: 13.

QHJ-02V13 comprises nucleic acid molecules encoding an IL-2/IL-15 chimeric polypeptide chain and an IL-15Rα-Fc fusion polypeptide chain, wherein the respective ORFs comprise SEQ ID NO: 15 and SEQ ID NO: 13. Alternatively, QHJ-02V13 comprises nucleic acid molecules encoding the same polypeptide chains, wherein the respective ORFs comprise nucleic acid sequences having at least 70% sequence identity to SEQ ID NO: 15 and SEQ ID NO: 13.

QHJ-02V15 comprises nucleic acid molecules encoding an IL-2/IL-15 chimeric polypeptide chain and an IL-15Rα-Fc fusion polypeptide chain, wherein the respective ORFs comprise SEQ ID NO: 15 and SEQ ID NO: 16. Alternatively, QHJ-02V15 comprises nucleic acid molecules encoding the same polypeptide chains, wherein the respective ORFs comprise nucleic acid sequences having at least 70% sequence identity to SEQ ID NO: 15 and SEQ ID NO: 16.

QHJ-02M02 comprises nucleic acid molecules encoding an IL-2/IL-15 chimeric polypeptide chain and an IL-15Rα-Fc fusion polypeptide chain, wherein the respective ORFs comprise SEQ ID NO: 15 and SEQ ID NO: 17. Alternatively, QHJ-02M02 comprises nucleic acid molecules encoding the same polypeptide chains, wherein the respective ORFs comprise nucleic acid sequences having at least 70% sequence identity to SEQ ID NO: 15 and SEQ ID NO: 17.
**SEQ ID NO: 12:** 
ATGTATCGGATGCAGCTGTTGTCCTGCATTGCTCTCTCATTGGCTCTGGTGACCAATA
GTGCTCCGACCTCCAGTAGTACGAAGAAGACACAGCTCAACTGGGTGAATGTAATAA
GTGATTTGAAAAAAATTGAAGATCTTATTCAATCTATGCATATTGATGCTACTTTATA
TACGGAAAGTGATGTTCACCCCAGTTGCAAAGTAACAGCAATGAAGTGCTTTCTCTT
GGAGTTACAAGTTATTTCACTTGAGTCCGGAGATGCAAGTATTCATGATACAGTAGA
AAATCTGATCATCCTAGCAAACGACAGTTTGTCTTCTAATGGGAATGTAACAGAATCT
GGATGCAAAGAATGTGAGGAACTGGAGGAAAAAAATATTAAAGAATTTTTGCAGAG
TTTTGTACATATTGTCCAAATGTTCATCAACACTTCTTAA.
**SEQ ID NO: 13** 
ATGGACAGACTTACTTCTTCATTCCTGCTCCTGATTGTCCCTGCGTACGTCTTGTCCAT
CACGTGCCCTCCCCCCATGTCCGTGGAACACGCAGACATCTGGGTCAAGAGCTACAG
CTTGTACTCCAGGGAGCGGTACATTTGTAACTCTGGTTTCAAGCGTAAAGCCGGCAC
GTCCAGCCTGACGGAGTGCGTGTTGAACAAGGCCACGAATGTCGCCCACTGGACAAC
 
CCCCAGTCTCAAATGTATTAGAGAGCCCAAATCTTGTGACAAAACTCACACATGCCC
ACCGTGCCCAGCACCTGAACTCCTGGGGGGACCGTCAGTCTTCCTCTTCCCCCCAAAA
CCCAAGGACACCCTCATGATCTCCCGGACCCCTGAGGTCACATGCGTGGTGGTGGAC
GTGAGCCACGAAGACCCTGAGGTCAAGTTCAACTGGTACGTGGACGGCGTGGAGGTG
CATAATGCCAAGACAAAGCCGCGGGAGGAGCAGTACAACAGCACGTACCGTGTGGT
CAGCGTCCTCACCGTCCTGCACCAGGACTGGCTGAATGGCAAGGAGTACAAGTGCAA
GGTCTCCAACAAAGCCCTCCCAGCCCCCATCGAGAAAACCATCTCCAAAGCCAAAGG
GCAGCCCCGAGAACCACAGGTGTACACCCTGCCCCCATCCCGGGATGAGCTGACCAA
GAACCAGGTCAGCCTGACCTGCCTGGTCAAAGGCTTCTATCCCAGCGACATCGCCGT
GGAGTGGGAGAGCAATGGGCAGCCGGAGAACAACTACAAGACCACGCCTCCCGTGC
TGGACTCCGACGGCTCCTTCTTCCTCTACAGCAAGCTCACCGTGGACAAGAGCAGGT
GGCAGCAGGGGAACGTCTTCTCATGCTCCGTGATGCATGAGGCTCTGCACAACCACT
ACACGCAGAAGAGCCTCTCCCTGTCTCCGGGTAAATAA.
**SEQ ID NO:14:** 
ATGTATCGGATGCAGCTGTTGTCCTGCATTGCTCTCTCATTGGCTCTGGTGACCAATA
GTGCTCCGACCTCCAGTAGTACGAAGAAGACACAGCTCAACTGGGTGAATGTAATAA
GTGATTTGAAAAAAATTGAAGATCTTATTCAATCTATGCATATTGATGCTACTTTATA
TACGGAAAGTGATGTTCACCCCAGTTGCAAAGTAACAGCAATGAAGTGCTTTCTCTT
GGAGTTACAAGTTATTTCACTTGAGTCCGGAGATGCAAGTATTCATGATACAGTAGA
AAATCTGATCATCCTAGCAAACGACAGTTTGTCTTCTAATGGGAATGTAACAGAATCT
GGATGCAAAGAATGTGAGGAACTGGAGGAAAAAAATATTAAAGAATTTTTGCAGAG
TTTTGTACATATTGTCCAAATGTTCATCAACACTTCTGACTACAAGGACGACGATGAT
AAGTAA.
**SEQ ID NO: 15:** 
ATGTACAGAATGCAGCTCTTGTCATGTATAGCACTGTCCCTCGCCCTCGTAACTAACA
GTGCCCCTACCAGCTCCAGTACTAAGAAAACACAGCTGAATTGGGTGAACGTCATCA
GCGATTTGAAAAAGATTGAAGACCTCATCCAGTCTATGCATATTGACGCTACCTTGTA
TACCGAATCTGACGTGCATCCAAGCTGTAAAGTGACCGCGATGAAGTGTTTTCTTCTG
GAGCTGCAAGTGATCTCTTTGGAATCAGGTGACGCTTCTATTCACGATACTGTGGAGA
ACCTCATTATCCTGGCTAACAACAGTCTGTCCTCAAATGGAAATGTGACCGAATCAG
GATGTAAGGAATGTGAAGAGCTGGAAGAGAAGAACATCAAAGAGTTTCTGCAAAGC
TTTGTGCACATCGTACAGATGTTTATCAACACTTCATAA.
**SEQ ID NO: 16:** 
ATGGACAGACTTACTTCTTCATTCCTGCTCCTGATTGTCCCTGCGTACGTCTTGTCCAT
CACGTGCCCTCCCCCCATGTCCGTGGAACACGCAGACATCTGGGTCAAGAGCTACAG
CTTGTACTCCAGGGAGCGGTACATTTGTAACTCTGGTTTCAAGCGTAAAGCCGGCAC
GTCCAGCCTGACGGAGTGCGTGTTGAACAAGGCCACGAATGTCGCCCACTGGACAAC
 
CCCCAGTCTCAAATGTATTAGAGAGCCCAAATCTTGTGACAAAACTCACACATGCCC
ACCGTGCCCAGCACCTGAATTCGAGGGGGGACCGTCAGTCTTCCTCTTCCCCCCAAA
ACCCAAGGACACCCTCATGATCTCCCGGACCCCTGAGGTCACATGCGTGGTGGTGGA
CGTGAGCCACGAAGACCCTGAGGTCAAGTTCAACTGGTACGTGGACGGCGTGGAGGT
GCATAATGCCAAGACAAAGCCGCGGGAGGAGCAGTACAACAGCACGTACCGTGTGG
TCAGCGTCCTCACCGTCCTGCACCAGGACTGGCTGAATGGCAAGGAGTACAAGTGCA
AGGTCTCCAACAAAGCCCTCCCAGCCAGCATCGAGAAAACCATCTCCAAAGCCAAAG
GGCAGCCCCGAGAACCACAGGTGTACACCCTGCCCCCATCCCGGGAGGAGATGACC
AAGAACCAGGTCAGCCTGACCTGCCTGGTCAAAGGCTTCTATCCCAGCGACATCGCC
GTGGAGTGGGAGAGCAATGGGCAGCCGGAGAACAACTACAAGACCACGCCTCCCGT
GCTGGACTCCGACGGCTCCTTCTTCCTCTACAGCAAGCTCACCGTGGACAAGAGCAG
GTGGCAGCAGGGGAACGTCTTCTCATGCTCCGTGATGCATGAGGCTCTGCACAACCA
CTACACGCAGAAGAGCCTCTCCCTGTCTCCGGGTAAATAA.
**SEQ ID NO: 17:**

ATGGACCGCCTGACTTCTTCTTTCCTTCTCCTTATCGTGCCCGCTTATGTTCTTTCAAT
CACATGTCCACCTCCCATGTCCGTCGAACATGCGGATATTTGGGTGAAATCCTACTCC
CTGTATTCAAGGGAACGGTATATTTGTAACTCCGGCTTTAAACGCAAGGCGGGAACT
TCTTCCCTGACTGAGTGCGTGCTCAACAAGGCTACCAACGTAGCTCATTGGACCACCC
CTTCCCTCAAATGCATCAGAGATCCTGCTCTGGTTCACCAACGGCCCAAGTCTTGCGA
CAAAACACATACCTGTCCCCCCTGTCCAGCTCCAGAACTTCTCGGAGGCCCATCTGTC
TTTCTGTTCCCACCTAAACCAAAAGATACTCTGATGATTTCAAGAACACCTGAAGTGA
CTTGTGTGGTCGTAGATGTCTCACATGAGGACCCCGAGGTTAAGTTTAACTGGTACGT
TGACGGCGTTGAGGTCCACAACGCTAAAACCAAACCAAGGGAGGAGCAGTACAATA
GCACGTATAGGGTGGTGTCAGTCCTTACAGTGCTGCATCAAGATTGGCTGAACGGAA
AGGAATACAAGTGCAAAGTGTCTAACAAGGCCCTGCCGGCTCCTATCGAGAAAACTA
TCAGTAAAGCGAAGGGTCAGCCCCGAGAGCCTCAGGTCTACACTCTTCCACCATCTC
GAGATGAGCTGACAAAAAATCAGGTTTCACTGACATGTCTGGTGAAAGGGTTCTACC
CTTCTGACATTGCAGTTGAGTGGGAAAGTAATGGTCAGCCTGAAAACAATTACAAAA
CAACACCGCCAGTGCTCGATTCTGATGGAAGCTTTTTCCTCTATTCAAAGCTGACAGT
GGACAAGTCAAGATGGCAGCAGGGAAACGTGTTCAGCTGCTCTGTGATGCACGAAGC
TCTGCATAACCACTACACCCAGAAAAGCCTGTCTCTTAGCCCCGGCAAGTAA.

In another aspect, the present invention provides expression vectors, each comprising a nucleic acid molecule encoding a corresponding polypeptide chain as described herein.

In some embodiments, the expression vector is a eukaryotic expression vector.

In more specific embodiments, the eukaryotic expression vector is a plasmid.

In another aspect, the present invention provides a host cell comprising a first expression vector comprising a nucleic acid molecule encoding the IL-2/IL-15 chimeric polypeptide chain; and a second expression vector comprising a nucleic acid molecule encoding the IL-15Rα-Fc fusion polypeptide chain.

In some embodiments, the host cell is a eukaryotic cell.

In more specific embodiments, the eukaryotic cell is selected from mammalian cells, insect cells, or yeast cells.

In particular embodiments, the mammalian cells are CHO cells or HEK293 cells.

In another aspect, the present invention provides the proteins, nucleic acid molecules, expression vectors, or host cells described herein for use in the manufacture of a medicament for treating cancer. In some embodiments, the cancer is selected from melanoma, lung cancer, acute granulocytic leukemia, chronic lymphocytic leukemia, acute lymphocytic leukemia, T-cell lymphoma, B-cell lymphoma, liver cancer, renal cancer, small cell lung cancer, non-small cell lung cancer, nasopharyngeal carcinoma, colorectal cancer, colon cancer, rectal cancer, pancreatic cancer, cervical cancer, ovarian cancer, breast cancer, bladder cancer, prostate cancer, acute myeloid leukemia, thyroid cancer, neuroblastoma, brain tumor, myeloma, and glioma.

In another aspect, the present invention provides the proteins, nucleic acid molecules, expression vectors, or host cells described herein for use in the manufacture of a medicament for inhibiting pulmonary metastasis of melanoma.

In another aspect, the present invention provides a pharmaceutical composition comprising the protein, the nucleic acid molecules, the expression vectors, or the host cell described herein.

In some embodiments, the pharmaceutical composition is for treating cancers.

In specific embodiments, the cancer is selected from melanoma, lung cancer, acute granulocytic leukemia, chronic lymphocytic leukemia, acute lymphocytic leukemia, T-cell lymphoma, B-cell lymphoma, liver cancer, renal cancer, small cell lung cancer, non-small cell lung cancer, nasopharyngeal carcinoma, colorectal cancer, colon cancer, rectal cancer, pancreatic cancer, cervical cancer, ovarian cancer, breast cancer, bladder cancer, prostate cancer, acute myeloid leukemia, thyroid cancer, neuroblastoma, brain tumor, myeloma, and glioma.

In some embodiments, the pharmaceutical composition comprises a pharmaceutically acceptable carrier.

Specifically, the pharmaceutically acceptable carrier is selected from excipients, buffers, emulsifiers, stabilizers, diluents, binders, preservatives, lubricants, pH regulators, cryoprotectants, and flavoring agents, or combinations thereof.

In some embodiments, the pharmaceutical composition is formulated in a dosage form suitable for gastrointestinal administration or non-gastrointestinal administration.

In some embodiments, the gastrointestinal dosage forms include tablets, powders, granules, solutions, capsules, emulsions, suspensions, and oils.

In some embodiments, the non-gastrointestinal dosage forms include injectable dosage forms, respiratory dosage forms, transdermal dosage forms, and mucosal dosage forms.

In specific embodiments, the injectable dosage forms include intravenous, intramuscular, subcutaneous, intradermal, or intracavitary injections.

In specific embodiments, the respiratory dosage forms include sprays, aerosols, or dry powders. In specific embodiments, the transdermal dosage forms include lotions, ointments, topical solutions, plasters, pastes, or patches.

In specific embodiments, the mucosal dosage forms include eye drops, nasal drops, ophthalmic ointments, sublingual tablets, or buccal films.

In specific embodiments, the body cavity dosage forms include suppositories, aerosols, effervescent tablets, drops, and pellet pills.

Preferably, the dosage form is an injectable dosage form.

More preferably, the injectable dosage form is a subcutaneous injection.

In some embodiments, the pharmaceutical composition is administered in combination with one or more anticancer agents, wherein the anticancer agents include Bacillus Calmette-Guérin (BCG), vemurafenib, dacarbazine, temozolomide, paclitaxel, cisplatin, carboplatin, and fotemustine.

### Beneficial effects:

The present invention provides the following advantages:
The protein exhibits increased expression levels.

The protein exhibits increased glycosylation.

The protein significantly reduces tumor volume and tumor area in melanoma-bearing mice, demonstrates a favorable therapeutic effect against melanoma, and effectively inhibits pulmonary metastasis of melanoma.

### Definition of Terms

Unless otherwise specified, the terms used herein have meanings commonly understood by a person skilled in the art in the relevant technical field.

As used herein, the term "IL-2" or "IL2", also known as T-cell growth factor, refers to a pleiotropic cytokine capable of:
(i) activating T cells and promoting their proliferation and cytokine production;
(ii) stimulating proliferation of natural killer (NK) cells, enhancing NK cell cytotoxic activity and cytokine production, and inducing the generation of lymphokine-activated killer (LAK) cells;
(iii) promoting B-cell proliferation and antibody secretion; and
(iv) activating macrophages.

The term "IL-2" encompasses IL-2 derived from any species, as well as variants or mutants thereof, including, without limitation, human IL-2, non-human mammalian IL-2, and non-mammalian IL-2. Exemplary non-human mammals include pig, rabbit, mouse, and non-human primates; exemplary non-mammals include chicken.

As used herein, the term "IL-15" or "IL15" refers to a pleiotropic cytokine capable of activating T cells, B cells, and natural killer (NK) cells, and promoting the proliferation and survival thereof; and activating, maintaining, and expanding CD8⁺ memory T cells. The term "IL-15" encompasses IL-15 derived from any species, as well as variants or mutants thereof, including, without limitation, human IL-15, non-human mammalian IL-15, and non-mammalian IL-15. Exemplary non-human mammals include pig, rabbit, mouse, and non-human primates; exemplary non-mammals include chicken.

As used herein, the term "IL-15Rα" refers to interleukin-15 receptor alpha, a receptor subunit capable of specifically binding IL-15 and mediating IL-15 signaling, from any species or a functional fragment thereof. The term includes full-length IL-15Rα as well as fragments retaining IL-15 binding activity, including, without limitation, the Sushi domain. The term "IL-15Rα" encompasses IL-15Rα derived from any species, including human IL-15Rα, non-human mammalian IL-15Rα, and non-mammalian IL-15Rα. Exemplary non-human mammals include pig, rabbit, monkey, ape, and mouse; exemplary non-mammals include chicken.

As used herein, the term "IL-15Rα-like protein" refers to a functional variant of IL-15Rα comprising one or more amino acid deletions, insertions, and/or substitutions, and retaining the ability to bind IL-15. Preferably, the IL-15Rα-like protein is derived from human IL-15Rα. More preferably, the IL-15Rα-like protein comprises an extracellular domain fragment of IL-15Rα, such as a fragment containing the Sushi domain, optionally with one or more amino acid modifications, including at the C-terminus, while retaining IL-15 binding activity and biological function.

As used herein, the term "IL-15Rα-Fc" refers to a fusion protein comprising an IL-15Rα moiety and an Fc region, wherein the IL-15Rα moiety comprises at least the Sushi domain capable of binding IL-15, and the Fc region comprises CH2 and CH3 domains. In some embodiments, the Fc region is derived from an immunoglobulin heavy chain constant region and is capable of mediating dimerization.

As used herein, the term "Fc region" refers to the constant region of an immunoglobulin heavy chain, in particular the C-terminal portion thereof, which lacks antigen-binding activity and mediates interactions with effector molecules and cells. The Fc region may be derived from human or non-human immunoglobulins and may include one or more constant domains selected from CH2, CH3, and CH4, optionally together with a hinge region.

Immunoglobulins are classified into IgA, IgD, IgE, IgG, and IgM, with certain classes further comprising subclasses, including IgG1, IgG2, IgG3, IgG4, IgA1, and IgA2. Preferably, the Fc region comprises at least a hinge region and CH2 and CH3 domains of IgG. More preferably, the Fc region comprises a hinge region, a CH2 domain, and a CH3 domain derived from IgG1.

As used herein, the term "sequence identity" refers to the percentage of identical amino acid residues or nucleic acid bases between a candidate sequence and a reference sequence following optimal alignment, wherein gaps may be introduced as necessary to achieve the best alignment. Sequence identity may be determined using methods known in the art, including publicly available sequence alignment algorithms such as BLAST or ALIGN, using default parameters unless otherwise specified.

As used herein, the term "vector" refers to a nucleic acid construct capable of transferring genetic material into a host cell, including DNA vectors (e.g., plasmids), RNA vectors, viral vectors, and other suitable replicons. Expression vectors comprise one or more polynucleotide sequences encoding a protein of interest operably linked to one or more regulatory elements that control transcription and/or translation and are suitable for expression of the protein in a host cell and/or integration into a host cell genome.

As used herein, the term "treatment" refers to any intervention, including, without limitation, surgical or pharmaceutical intervention, aimed at preventing, alleviating, ameliorating, or inhibiting a disease or condition, such as cancer, including melanoma. Treatment may result in one or more beneficial effects, including, without limitation, alleviation of one or more symptoms, reduction in disease severity, stabilization of disease state, delay or slowing of disease progression, and partial or complete remission.

As used herein, the term "pharmaceutical composition" refers to a composition comprising at least one active agent according to the present invention together with one or more pharmaceutically acceptable carriers, excipients, and/or diluents, wherein the composition is suitable for administration to a subject.

### Brief Description of the Drawings

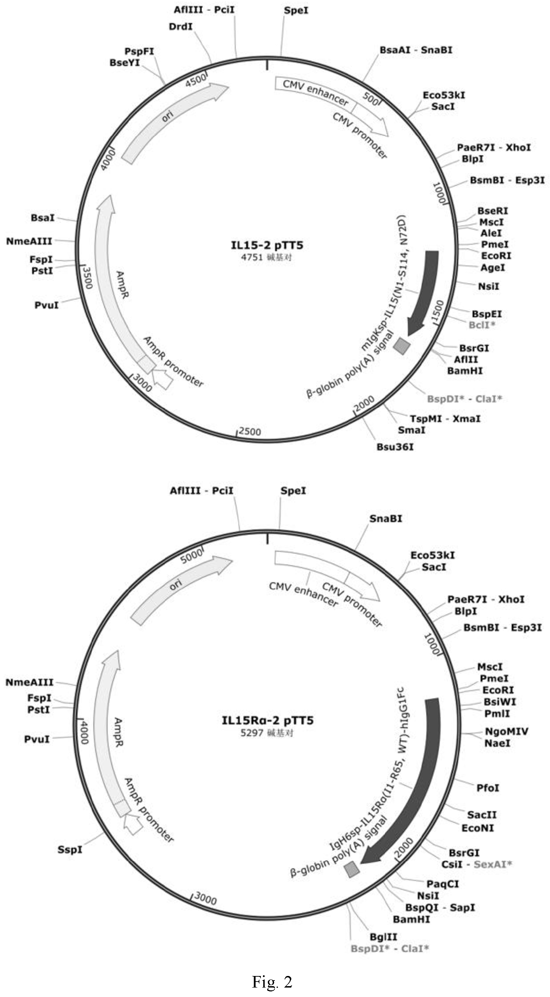

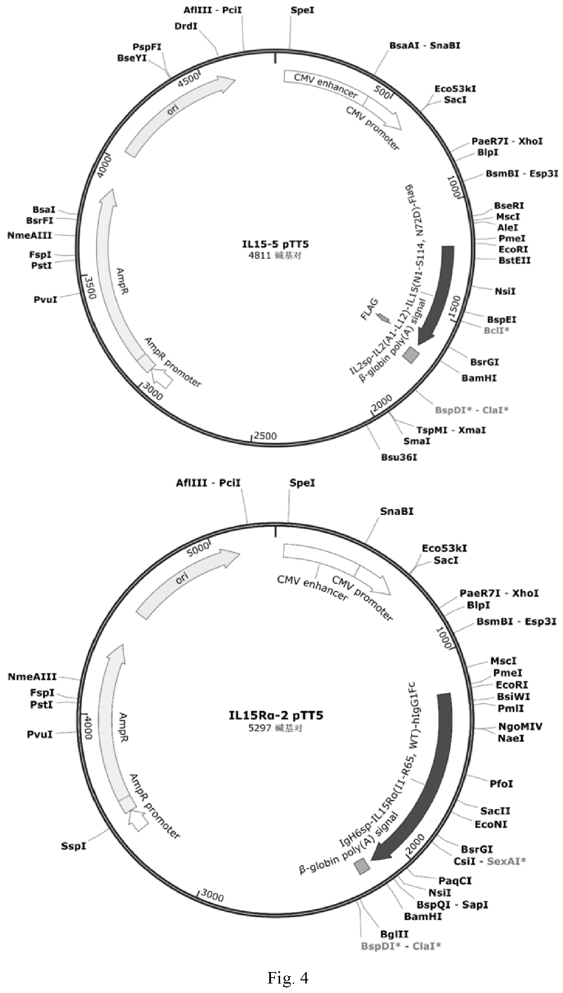

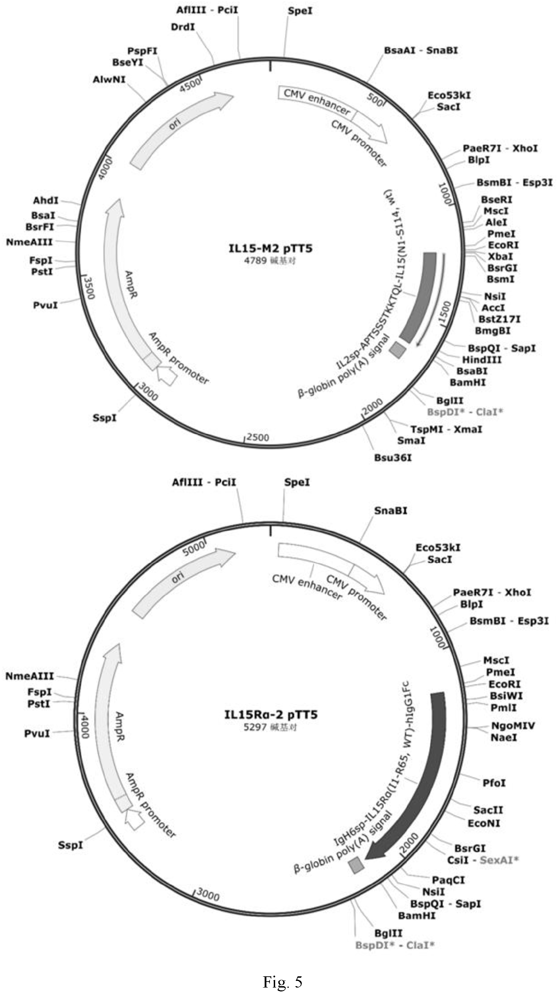

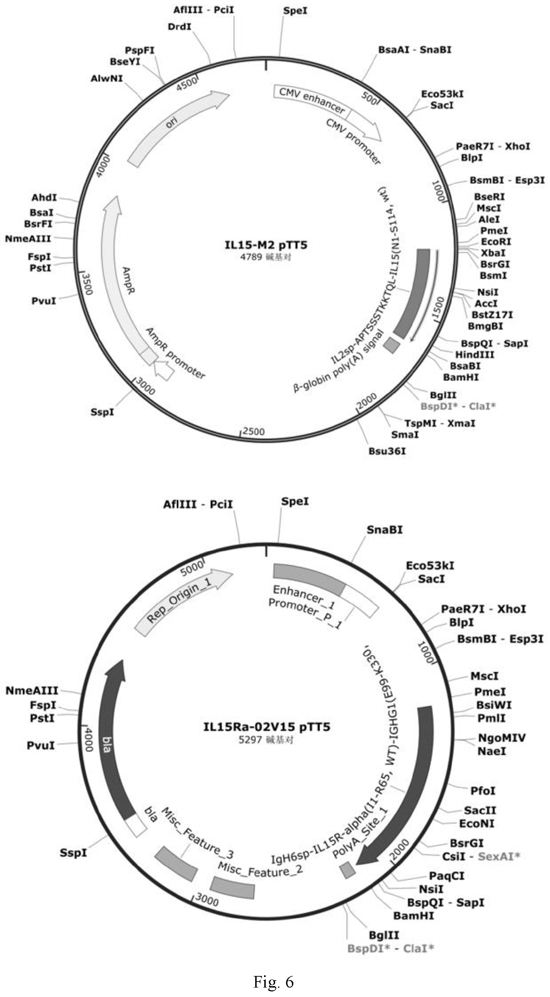

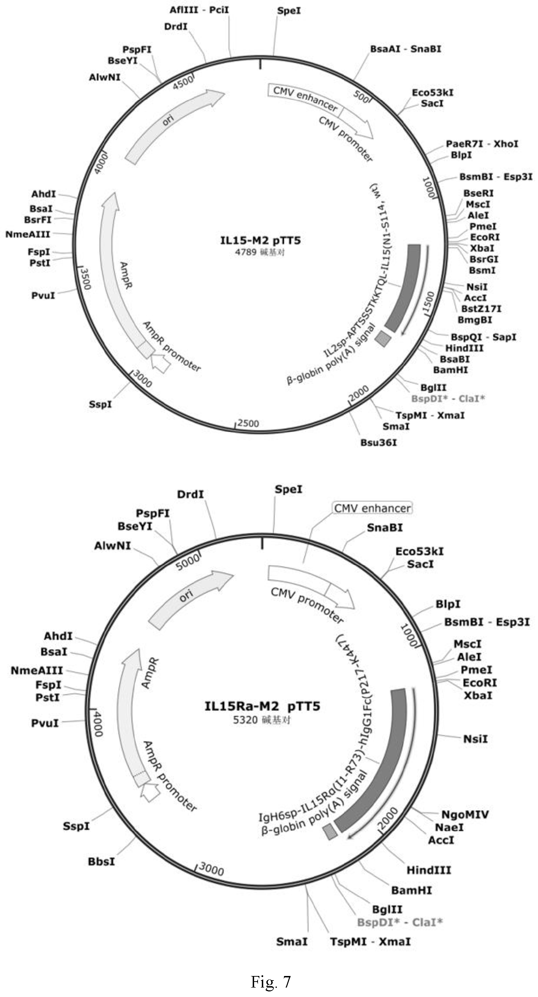

The three Chinese characters appearing on plasmid maps in the figures 2-7 mean "base pairs" in English.

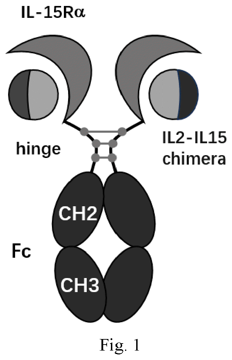

FIG. 1 is a schematic diagram of a protein of the present invention, which is a homodimeric complex formed by dimerization of two heterodimeric units. Each heterodimeric unit comprises an IL-2/IL-15 chimeric polypeptide chain and an IL-15Rα-Fc fusion polypeptide chain. The IL-2/IL-15 chimeric polypeptide comprises an N-terminal polypeptide segment derived from IL-2 fused to a full-length IL-15 polypeptide or a variant thereof. The IL-15Rα-Fc fusion polypeptide comprises the Sushi domain of IL-15Rα and an Fc region comprising CH2 and CH3 domains. Within each heterodimeric unit, the IL-2/IL-15 chimeric polypeptide chain and the IL-15Rα-Fc fusion polypeptide chain associate via ligand-receptor binding interactions. Two heterodimeric units form a homodimeric complex through interactions between the two Fc regions including disulfide bonds in the hinge region and interactions between the CH3 domains.
FIG. 2 shows two plasmids encoding the IL-15 polypeptide and the IL-15Rα-Fc fusion polypeptide, respectively, for expression of the reference protein QHJ-02V02 (N-803).
FIG. 3 shows two plasmids encoding the IL-2/IL-15 chimeric polypeptide and the IL-15Rα-Fc fusion polypeptide, respectively, for expression of QHJ-02V03.
FIG. 4 shows two plasmids encoding the IL-2/IL-15 chimeric polypeptide and the IL-15Rα-Fc fusion polypeptide, respectively, for expression of QHJ-02V05.
FIG. 5 shows two plasmids encoding the IL-2/IL-15 chimeric polypeptide and the IL-15Rα-Fc fusion polypeptide, respectively, for expression of QHJ-02V13.
FIG. 6 shows two plasmids encoding the IL-2/IL-15 chimeric polypeptide and the IL-15Rα-Fc fusion polypeptide, respectively, for expression of QHJ-02V15.
FIG. 7 shows two plasmids encoding the IL-2/IL-15 chimeric polypeptide and the IL-15Rα-Fc fusion polypeptide, respectively, for expression of QHJ-02M02.
FIG. 8 shows an SDS-PAGE analysis of QHJ-02V02 protein following Protein A affinity chromatography, wherein M denotes a molecular weight marker; S denotes culture supernatant; FT denotes flow-through; and E_{0.1 M Gly} denotes the elution fraction obtained using 0.1 M glycine.
FIG. 9 shows an SDS-PAGE analysis of QHJ-02V02 protein after deglycosylation using PNGase F (N-glycosidase F).
FIG. 10 shows an SDS-PAGE analysis of QHJ-02V03 protein following Protein A affinity chromatography.
FIG. 11 shows an SDS-PAGE analysis of QHJ-02V03 protein after deglycosylation using PNGase F.
FIG. 12 shows an SDS-PAGE analysis of QHJ-02V05 protein following Protein A affinity chromatography.
FIG. 13 shows a chromatogram of QHJ-02V02 obtained using Q HP (5 mL) ion-exchange chromatography.
FIG. 14 shows an SDS-PAGE analysis of QHJ-02V02 obtained following Q HP ion-exchange chromatography.
FIG. 15 shows a chromatogram of QHJ-02V02 Peak 1 following size-exclusion chromatography (SEC) using a Superdex 200 10/300 column.
FIG. 16 shows an SDS-PAGE analysis of QHJ-02V02 Peak 1 following SEC.
FIG. 17 shows a chromatogram of QHJ-02V02 Peak 2 following SEC under the same conditions as in FIG. 15.
FIG. 18 shows an SDS-PAGE analysis of QHJ-02V02 Peak 2 following SEC.
FIG. 19 shows an SDS-PAGE analysis of purified QHJ-02V02.
FIG. 20 shows an SEC chromatogram of purified QHJ-02V02 Peak 1.
FIG. 21 shows an SEC chromatogram of purified QHJ-02V02 Peak 2.
FIG. 22 shows an intact mass spectrum of QHJ-02V02 Peak 1.
FIG. 23 shows an intact mass spectrum of QHJ-02V02 Peak 2.
FIG. 24 shows a chromatogram of QHJ-02V03 obtained under the same Q HP ion-exchange chromatography conditions as in FIG. 13.
FIG. 25 shows an SDS-PAGE analysis of QHJ-02V03 obtained following Q HP ion-exchange chromatography.
FIG. 26 shows a chromatogram of QHJ-02V03 Peak 1 following SEC under the same conditions as in FIG. 15.
FIG. 27 shows an SDS-PAGE analysis of QHJ-02V03 Peak 1 following SEC.
FIG. 28 shows a chromatogram of QHJ-02V03 Peak 2 following SEC under the same conditions as in FIG. 26.
FIG. 29 shows an SDS-PAGE analysis of QHJ-02V03 Peak 2 following SEC.
FIG. 30 shows an SDS-PAGE analysis of purified QHJ-02V03.
FIG. 31 shows an SEC chromatogram of QHJ-02V03 Peak 1.
FIG. 32 shows an SEC chromatogram of QHJ-02V03 Peak 2.
FIG. 33 shows an intact mass spectrum of QHJ-02V03 Peak 1.
FIG. 34 shows an intact mass spectrum of QHJ-02V03 Peak 2.
FIG. 35 shows Mo7e cell proliferation induced by IL-15 (Acro, Cat. No.: IL5-H5215).
FIG. 36 shows Mo7e cell proliferation induced by QHJ-02V02 Peak 1.
FIG. 37 shows Mo7e cell proliferation induced by QHJ-02V02 Peak 2.
FIG. 38 shows Mo7e cell proliferation induced by QHJ-02V03 Peak 1.
FIG. 39 shows Mo7e cell proliferation induced by QHJ-02V03 Peak 2.
FIG. 40 shows tumor volume curves over time in a B16F10 tumor model for groups including a negative control (PBS), a positive control (IL-15, 2 µg), and reference protein groups (QHJ-02V02 Peak 1 and Peak 2 at 5 µg and 15 µg).
FIG. 41 shows a statistical analysis of tumor volume reduction at the experimental endpoint for the treatment groups described in FIG. 40.
FIG. 42 shows average body weight curves over time in a B16F10 melanoma lung metastasis model for groups including a negative control (PBS), a positive control (IL-15, 2 µg), reference protein groups (QHJ-02V02 at 5 µg and 15 µg), and groups treated with proteins of the present invention (QHJ-02V03 at 5 µg and 15 µg).
FIG. 43 shows average body temperature curves over time obtained from the same experiment as in FIG. 42.
FIG. 44 shows the average number of pulmonary metastatic lesions at the experimental endpoint, obtained from the same experiment as in FIG. 42. Each treatment group was statistically compared with the negative control group (*P < 0.05, **P ≤ 0.01, ***P ≤ 0.001).
FIG. 45 shows photographs of pulmonary metastatic lesions in the seven mice of the negative control group. The black nodules on the lungs represent metastatic lesions. The photographs were taken from both the ventral and dorsal sides of each lung.
FIG. 46 shows photographs for the positive control group (IL-15, 2 µg), obtained under the same imaging conditions as in FIG. 45.
FIG. 47 shows photographs for the reference group (QHJ-02V02, 5 µg), obtained under the same imaging conditions as in FIG. 45.
FIG. 48 shows photographs for the reference group (QHJ-02V02, 15 µg), obtained under the same imaging conditions as in FIG. 45.
FIG. 49 shows photographs for the group treated with proteins of the present invention (QHJ-02V03 at 5 µg), obtained under the same imaging conditions as in FIG. 45.
FIG. 50 shows photographs for the group treated with proteins of the present invention (QHJ-02V03 at 15 µg), obtained under the same imaging conditions as in FIG. 45.
FIG. 51 shows an SDS-PAGE analysis of QHJ-02V13 following Protein A affinity chromatography. The left panel shows proteins under reducing conditions, and the right panel shows proteins under non-reducing conditions.
FIG. 52 shows an SDS-PAGE analysis of QHJ-02V13 following SEC using a Superdex 200 10/300 column, where fractions 2-8 were collected and analyzed. The corresponding chromatogram indicating fractions 2-8 is shown in the bottom panel.
FIG. 53 shows an SDS-PAGE analysis of QHJ-02V15 following Protein A affinity chromatography under the same conditions as in FIG. 51.
FIG. 54 shows an SDS-PAGE analysis of QHJ-02V15 following SEC using a Superdex 200 10/300 column, where fractions 2-8 were collected and analyzed under the same conditions as in FIG. 52.
FIG. 55 shows an SDS-PAGE analysis of QHJ-02M02 following Protein A affinity chromatography. The left lanes show proteins under reducing conditions, and the right lanes show proteins under non-reducing conditions.
FIG. 56 shows a chromatogram of QHJ-02M02 following SEC using a Superdex 200 10/300 column, where fractions 1-8 were collected and analyzed. The corresponding SDS-PAGE analysis of fractions 1-8 is shown in the bottom panel.
FIG. 57 shows Mo7e cell proliferation induced by QHJ-02M02.
FIG. 58 shows tumor volume curves over time in a B16F10 tumor model for groups including a negative control (PBS), a positive control (IL-15, 2 µg), and groups treated with proteins of the present invention, including QHJ-02V03, QHJ-02V13, and QHJ-02V15 (each at 2 µg and 5 µg).
FIG. 59 shows statistical analysis of tumor volume reduction from the same experiment as in FIG. 58. Each treatment group was statistically compared with the negative control group (*P < 0.05, **P ≤ 0.01, ***P ≤ 0.001).

### Examples

The present invention is further described below by way of non-limiting embodiments to facilitate understanding of the technical features and effects of the invention. These embodiments are provided for illustrative purposes only and are not intended to limit the scope of the invention as defined by the appended claims. Modifications and variations of the embodiments described herein may be made by those skilled in the art without departing from the scope of the appended claims. Unless otherwise indicated, the methods used in the following embodiments are conventional, the equipment used is standard laboratory equipment, and the materials and reagents are commercially available from known sources.

### General Embodiments of the Proteins of the Invention

A schematic structural representation of the proteins of the present invention is shown in FIG. 1.

In some embodiments, the proteins each comprises an IL-2/IL-15 chimeric polypeptide chain and an IL-15Rα-Fc fusion polypeptide chain to form a heterodimeric unit, wherein the IL-2/IL-15 chimeric polypeptide comprises an IL-2 fragment fused to an IL-15 polypeptide or a variant thereof, and the IL-15Rα-Fc fusion polypeptide comprises the sushi domain of IL-15Rα fused to an Fc region; wherein two heterodimeric units associate via Fc-mediated dimerization to form a homodimeric complex.

In some embodiments, the proteins may be produced by co-expression in a host cell via co-transfection of two expression vectors, wherein one expression vector encodes the IL-2/IL-15 chimeric polypeptide chain, and the other expression vector encodes the IL-15Rα-Fc fusion polypeptide chain.

Table 1 lists exemplary signal peptide sequences suitable for expression of the proteins of the present invention.

**Table 1: Signal Peptide Sequences**

| **SEQ ID NO.** | **Signal Peptide Name** | **Signal Peptide Sequence** |
|---|---|---|
| SEQ ID NO: 1 | Human Ig κ-chain V-III signal peptide | MDMRVPAQLLGLLLLWLRGARC |
| SEQ ID NO: 2 | Mouse Ig kappa signal peptide | METDTLLLWVLLLWVPGSTG |
| SEQ ID NO: 3 | IgH-6 signal peptide | MDRLTSSFLLLIVPAYVLS |
| SEQ ID NO: 4 | Human IL-2 signal peptide | MYRMQLLSCIALSLALVTNS |

A reference protein used herein is N-803 (designated herein as QHJ-02V02), comprising an IL-15 polypeptide (SEQ ID NO: 5) and an IL-15Rα-Fc fusion polypeptide (SEQ ID NO: 6); and the corresponding nucleic acid sequences encoding IL-15 and IL-15Rα-Fc are SEQ ID NO: 18 and SEQ ID NO: 13, respectively.
**SEQ ID NO: 5:** 
NWVNVISDLKKIEDLIQSMHIDATLYTESDVHPSCKVTAMKCFLLELQVISLESGDASIHD
TVENLIILANDSLSSNGNVTESGCKECEELEEKNIKEFLQSFVHIVQMFINTS.
**SEQ ID NO: 18:**

ATGGAGACAGACACACTCCTGTTATGGGTACTGCTGCTCTGGGTTCCAGGTTCCACCG
GTAACTGGGTGAATGTAATAAGTGATTTGAAAAAAATTGAAGATCTTATTCAATCTA
TGCATATTGATGCTACTTTATATACGGAAAGTGATGTTCACCCCAGTTGCAAAGTAAC
AGCAATGAAGTGCTTTCTCTTGGAGTTACAAGTTATTTCACTTGAGTCCGGAGATGCA
AGTATTCATGATACAGTAGAAAATCTGATCATCCTAGCAAACGACAGTTTGTCTTCTA

ATGGGAATGTAACAGAATCTGGATGCAAAGAATGTGAGGA
ACTGGAGGAAAAAAATATTAAAGAATTTTTGCAGAGTTTTGTACATA
TTGTCCAAATGTTCATCAACACTTCTTAA.

In some embodiments, the proteins of the present invention comprise the following compositions: QHJ-02V03, comprising an IL-2/IL-15 chimeric polypeptide (SEQ ID NO: 7) and an IL-15Rα-Fc fusion polypeptide (SEQ ID NO: 6);
QHJ-02V05, comprising an IL-2/IL-15 chimeric polypeptide (SEQ ID NO: 8) and an IL-15Rα-Fc fusion polypeptide (SEQ ID NO: 6);
QHJ-02V13, comprising an IL-2/IL-15 chimeric polypeptide (SEQ ID NO: 9) and an IL-15Rα-Fc fusion polypeptide (SEQ ID NO: 6);
QHJ-02V15, comprising an IL-2/IL-15 chimeric polypeptide (SEQ ID NO: 9) and an IL-15Rα-Fc fusion polypeptide (SEQ ID NO: 10); and
QHJ-02M02, comprising an IL-2/IL-15 chimeric polypeptide (SEQ ID NO: 9) and an IL-15Rα-Fc fusion polypeptide (SEQ ID NO: 11).

In some embodiments, the nucleic acid sequences encoding the corresponding proteins of the present invention are as follows:
QHJ-02V03 includes:
   (1) IL-2/IL-15 chimera: SEQ ID NO: 12;
   (2) IL-15Rα-Fc fusion: SEQ ID NO: 13.
QHJ-02V05 includes:
   (1) IL-2/IL-15 chimera: SEQ ID NO: 14;
   (2) IL-15Rα-Fc fusion: SEQ ID NO: 13.
QHJ-02V13 includes:
   (1) IL-2/IL-15 chimera: SEQ ID NO: 15;
   (2) IL-15Rα-Fc fusion: SEQ ID NO: 13.
QHJ-02V15 includes:
   (1) IL-2/IL-15 chimera: SEQ ID NO: 15;
   (2) IL-15Rα-Fc fusion: SEQ ID NO: 16.
QHJ-02M02 includes:
   (1) IL-2/IL-15 chimera: SEQ ID NO: 15;
   (2) IL-15Rα-Fc fusion: SEQ ID NO: 17.

The sequences described herein are exemplary, and variants, derivatives, and functionally equivalent sequences having substantially the same biological activity are also contemplated. The plasmid maps of the reference protein are shown in FIG. 2.

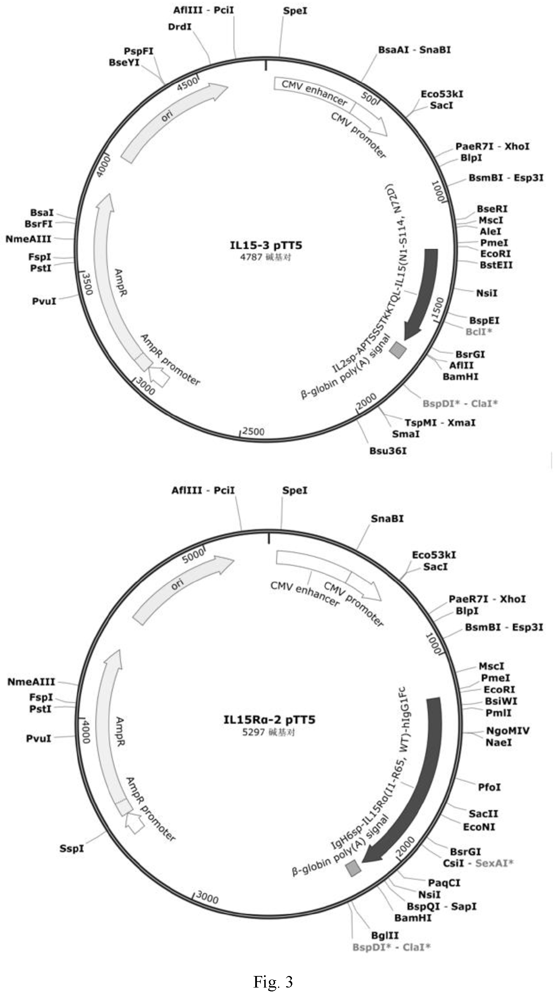

In some embodiments, the plasmid maps corresponding to the proteins of the present invention are shown in FIGS. 3-7, respectively, corresponding to QHJ-02V03, QHJ-02V05, QHJ-02V13, QHJ-02V15, and QHJ-02M02.

### Example 1: Vector construction, Cell culture, and Transient expression Vector Construction

As shown in FIGS. 2-7, DNA fragments encoding the IL-15 polypeptide or IL-2/IL-15 chimeric polypeptides, and IL-15Rα-Fc fusion polypeptides were synthesized (GenScript, Nanjing, China). All DNA fragments were amplified by polymerase chain reaction (PCR) using PrimeSTAR Max DNA Polymerase (Takara, Tokyo, Japan) according to the manufacturer's instructions and subsequently inserted into the plasmid vectors (as shown in Figures 2-7) using a Seamless Cloning Kit (Beyotime, China), in accordance with the manufacturer's instructions. After sequence verification, EndoFree Plasmid Maxi (Qiagen, Germany) kits were used to prepare the plasmids. The plasmids were stored at -20°C until use.

The PCR primers used for DNA amplification are listed in Table 2

**Table 2: PCR Primers Used for DNA Amplification for the corresponding protein**

| Corresponding protein | Primer Sequence (SEQ ID NO) |
|---|---|
| QHJ-02V02 (IL-15) | AACGGATCTCTAGCGAATTCGCCACCATGGAGACAGACACACTCCTG TTATGGGTACTG (SEQ ID NO: 19) |
| | ACCGGTGGAACCTGGAACCCAGAGCAGCAGTACCCATAACAGGAGT GTG (SEQ ID NO: 20) |
| | TGGGTTCCAGGTTCCACCGGTAACTGGGTGAATGTAATAAGT (**SEQ ID NO: 21**) |
| | GAGGTCGAGGTCGGGGGATCCTTAAGAAGTGTTGATGAACATTTG (**SEQ ID NO: 22**) |
| QHJ-02V03 (IL-2/IL-15 chimera) | AACGGATCTCTAGCGAATTCGCCACCATGTATCGGATGCAGCTGTTG (**SEQ ID NO: 23**) |
| | GAGGTCGAGGTCGGGGGATCCTTAAGAAGTGTTGATGAACATTTG (**SEQ ID NO: 24**) |
| QHJ-02V05 (IL-2/IL-15 chimera) | AACGGATCTCTAGCGAATTCGCCACCATGTATCGGATGCAGCTGTTG (**SEQ ID NO: 25**) |
| | CTTATCATCGTCGTCCTTGTAGTCAGAAGTGTTGATGAACATTTG (**SEQ ID NO: 26**) |
| | GAGGTCGAGGTCGGGGGATCCTTACTTATCATCGTCGTCCTTGTAGTC (**SEQ ID NO: 27**) |
| QHJ-02V13 (IL-2/IL-15 chimera) | CCACTCCCAGGTCCAAGTTTAAACGGATCTCTAGCGAATTCATGTACA GAATGCA (SEQ ID NO: 30) |
| | TAAATTTCCTTTATTAGCCAGAGGTCGAGGTCGGGGATCCTCAGCTT GTGTTGATGAACATC (SEQ ID NO: 31) |
| QHJ-02V02 (IL-15Rα Fc) | AACGGATCTCTAGCGAATTCGCCACCATGGACAGACTTACTTCTT (**SEQ ID NO: 28**) |
| | GAGGTCGAGGTCGGGGGATCCTTATTTACCCGGAGACAGGGA (**SEQ ID NO: 29**) |
| QHJ-02V15 (IL-15Rα Fc) | AACGGATCTCTAGCGAATTCGCCACCATGGACAGACTTACTTCTT (**SEQ ID NO: 32**) |
| | GAGGTCGAGGTCGGGGGATCCTTATTTACCCGGAGACAGGGAGAG (**SEQ ID NO:33**) |
| QHJ-02M02 (IL-15Rα Fc) | CCACTCCCAGGTCCAAGTTTAAACGGATCTCTAGCGAATTCATGGAC AGACTGACAAGCAG (SEQ ID NO:34) |
| | TAAATTTCCTTTATTAGCCAGAGGTCGAGGTCGGGGGATCCTCACTTG CCGGGGCTCAG (SEQ ID NO:35) |

### Cell Culture and Transient Expression

(1) Suspension-culture Expi293F cells (Thermo Fisher Scientific) according to manufacter's instruction. Briefly, Cells in flasks were cultured in BalanceCD 293 medium (CELL-WISE) supplemented with penicillin/streptomycin in a humidified incubator at 37°C with 8% CO₂.
(2) Prepare the transfection mixture. The IL-2/IL-15 chimera plasmid and the IL-15Rα-Fc plasmid were diluted in pre-warmed (37 °C) Opti-MEM medium (Gibco) and mixed to a total amount of 100 µg per 100 mL of cell culture, at a weight ratio of 1:1 or 5:1 (IL-2/IL-15 chimera plasmid : IL-15Rα-Fc plasmid), followed by the addition of PEI solution. The mixture was gently mixed, incubated at room temperature for 20 minutes, and was then ready for transfection.
(3) Prepare the cell culture for transfection by increasing its volume to 0.1 L so that the culture reaches a cell density of 2.8 × 10⁶ cells/mL, then co-transfect the culture by adding the transfection mixture from step 2.
(4) Add transfection enhancer. Approximately 20 hours post-transfection, 2 mL of transfection enhancer was added while gently swirling the flask. The enhancer comprised vGlucos (400 g/L) and vVPA (100 g/1.5 L) at a volume ratio of 7:1.
   The cells were cultured for approximately 5 days after addition of transfection enhancer to allow protein production.
(5) The cell culture was harvested by centrifugation at 2000 rpm and 4 °C to remove most cells, followed by a second centrifugation at 8000 rpm to remove residual cell debris. The supernatant containing the expressed protein was collected and used directly for subsequent purification. Expression of the target proteins was confirmed by SDS-PAGE.

### Example 2: Measurement of Protein Expression Levels for QHJ-02V02, QHJ-02V03, and QHJ-02V05

(1) Pre-equilibrated 0.5 ml Protein A resin (1× PBS, pH 7.4, 4°C) was mixed with about 100 mL of supernatant (S) from Example 1. The mixture was incubated for an hour to allow sufficient target protein binding by Protein A.
(2) The mixture was centrifuged at 1000 rpm for 5 minutes, and the supernatant was collected as the flow-through (FT). The resin was then washed twice with 1 mL of 1× PBS (pH 7.4).
(3) The bound protein was eluted with 1 mL elution buffer (0.1 M glycine, pH 3.0) 3X.

Each eluted fraction was immediately neutralized to physiological pH by adding 100 µL of neutralization buffer (1 M Tris, pH 8.0) per 1 mL of elution buffer.

The protein yields are summarized in Table 3.

**Table 3: Protein Yields**

| Protein | Plasmid Ratio (IL-15 or IL-2/IL-15):IL-15Rα-Fc ) | Elution Volume (mL) | Protein Concentration (mg/mL) | Yield (mg/L) |
|---|---|---|---|---|
| QHJ-02V02 | 1:1 | 6 mL | 0.32 | 20 |
| | 5:1 | | 0.39 | 23 |
| QHJ-02V03 | 1:1 | 6 mL | 0.65 | 39 |
| | 5:1 | | 0.42 | 25 |
| QHJ-02V05 | 1:1 | 6 mL | 0.58 | 35 |
| | 5:1 | | 0.40 | 24 |

### Results and Analysis

From the data presented in Table 3, the following observations can be made:
Increasing the plasmid co-transfection ratio from 1:1 to 5:1 did not result in a significant increase in protein expression levels.

The protein expression level of QHJ-02V03 is approximately two-fold that of QHJ-02V02 under comparable conditions.

Comparison of QHJ-02V05 with QHJ-02V03 indicates that the presence of a C-terminal FLAG tag has only a minor effect on protein yield.

### Example 3: Characterization of QHJ-02V02, QHJ-02V03, and QHJ-02V05 by gel electrophoresis

### QHJ-02V02

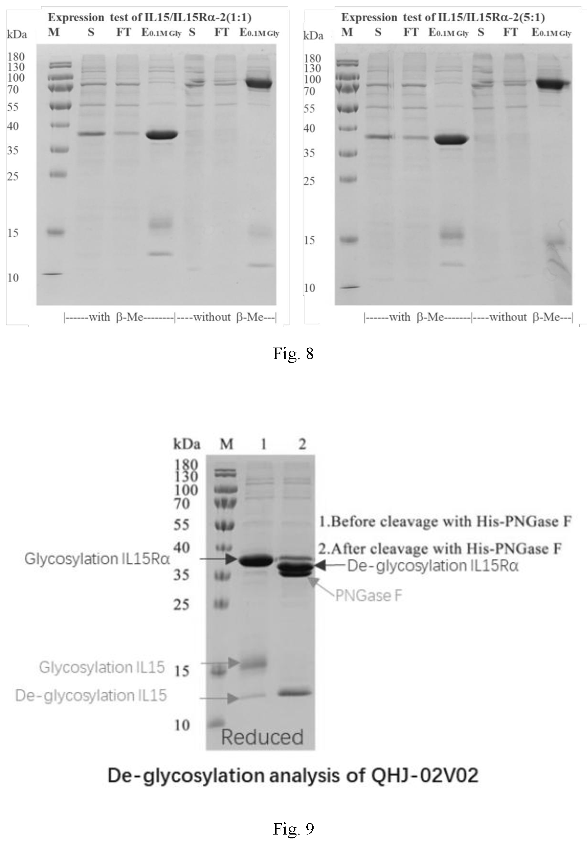

Purified proteins obtained after Protein A affinity chromatography were analyzed by 12% SDS-PAGE under reducing and non-reducing conditions (FIG. 8).

Under reducing conditions (β-mercaptoethanol), three bands were observed at approximately 38 kDa, 16 kDa, and 13 kDa. The 38 kDa band corresponds to the glycosylated IL-15Rα (I1-R65)-Fc (CH2-CH3) chain (theoretical unglycosylated molecular weight: 33.4 kDa). The 16 kDa band corresponds to glycosylated IL-15, and the 13 kDa band corresponds to unglycosylated IL-15 (theoretical molecular weight: 12.8 kDa).

Under non-reducing conditions, a band at approximately 80 kDa was observed, corresponding to the dimer of the glycosylated IL-15Rα-Fc chain.

(4) Following PNGase F treatment (FIG. 9), the IL-15Rα-Fc band shifted from ~36 kDa to ~34 kDa, and the IL-15 band shifted from ~16 kDa to ~13 kDa.

### QHJ-02V03

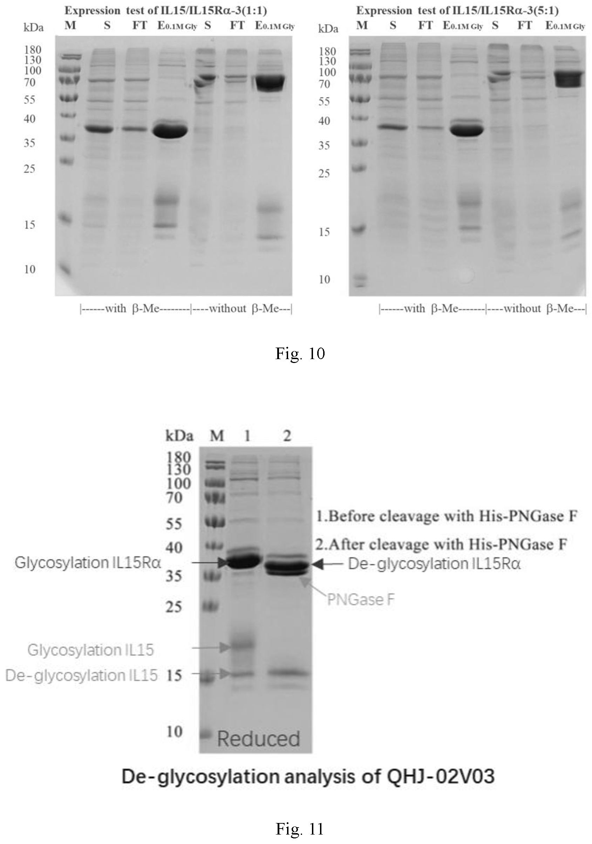

Results of 12% SDS-PAGE are shown in FIG. 10.

Under reducing conditions, four bands were observed at approximately 38 kDa, 19 kDa, 15 kDa, and 14 kDa. The 38 kDa band corresponds to the glycosylated IL-15Rα-Fc chain. The IL-2/IL-15 chimeric chain exhibited three forms: highly glycosylated (~19 kDa), moderately glycosylated (~15 kDa), and slightly or minimally glycosylated (~14 kDa).

After PNGase F treatment (FIG. 11), three bands were observed at ~36 kDa, 16 kDa, and 14 kDa. The 36 kDa band corresponds to IL-15Ra~Fc, the 14 kDa band to the deglycosylated IL-2/IL-15 chain, and the 16 kDa band to a partially deglycosylated form.

In contrast to QHJ-02V02 (FIG. 9), where glycosylated IL-15 is largely converted to the unglycosylated form, the IL-2/IL-15 chimeric chain is predominantly converted to partially deglycosylated species. This is consistent with PNGase F removing accessible N-linked glycans while leaving additional glycosylation not removed under these conditions, including glycosylation associated with the introduced IL-2-derived sequence. These results indicate increased and more complex glycosylation relative to IL-15 alone.

### QHJ-02V05

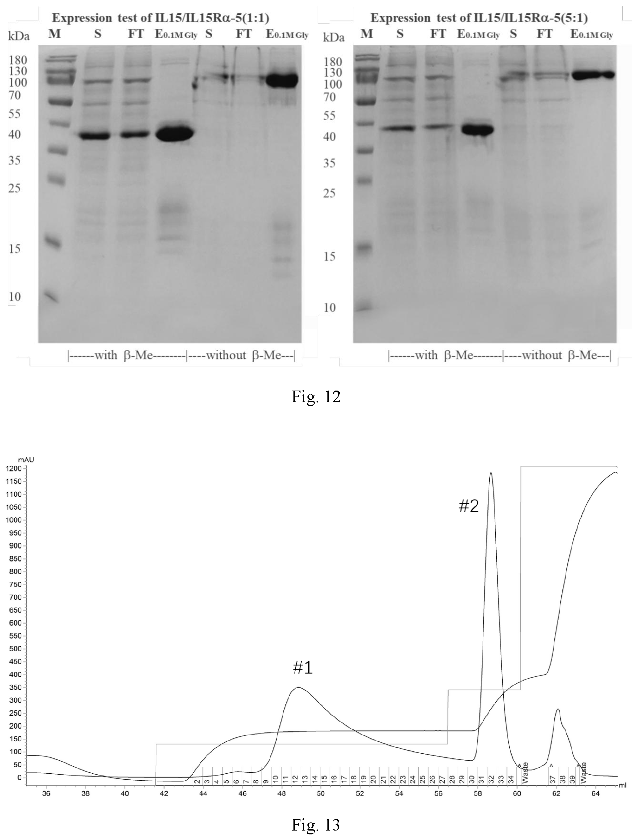

Results of 12% SDS-PAGE are shown in FIG. 12.

Under reducing conditions, a band at ~38 kDa and a cluster of bands between 15-20 kDa were observed. The 38 kDa band corresponds to the glycosylated IL-15Rα-Fc chain, while the 15-20 kDa region corresponds to multiple glycoforms of the IL-2/IL-15 chimeric chain.

This pattern is consistent with a higher degree of glycosylation compared to QHJ-02V02 and is similar to that observed for QHJ-02V03.

Collectively, these results indicate that incorporation of the IL-2 sequence results in multiple glycoforms and increased glycosylation relative to IL-15 alone.

### Example 4: Further Purification and Characterization of QHJ-02V02 and QHJ-02V03

Further purification was performed based on literature methods with modifications. (Han, K. P., Zhu, X., Liu, B., Jeng, E., Kong, L., & Yovandich, J. L., et al., *Cytokine*, 2011, 56(3): 804-810). Protein A-purified material was subjected to anion exchange chromatography (Q HP), followed by size exclusion chromatography (SEC).

The buffers used for the Q HP column were as follows:
Buffer A: 20 mM Tris, pH 8.0;
Buffer B: 20 mM Tris, 1 M NaCl, pH 8.0.

The buffer used for SEC was phosphate-buffered saline (PBS), pH 7.4.

### QHJ-02V02

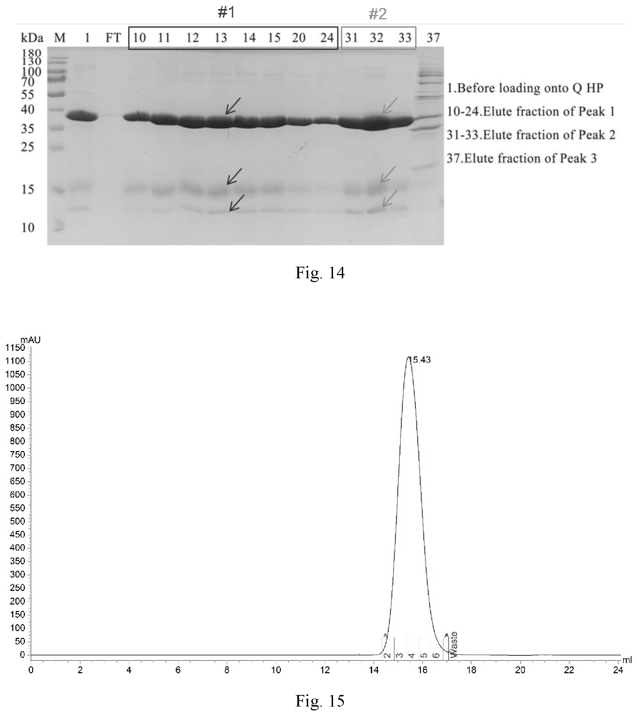

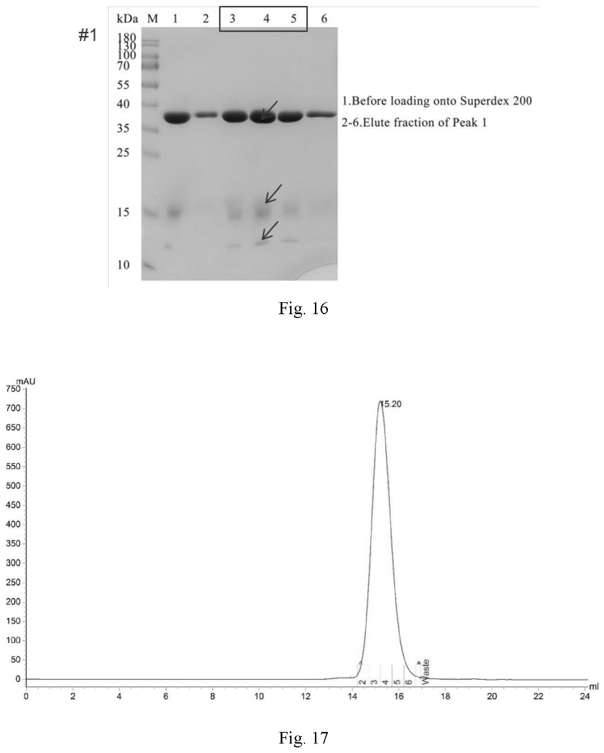

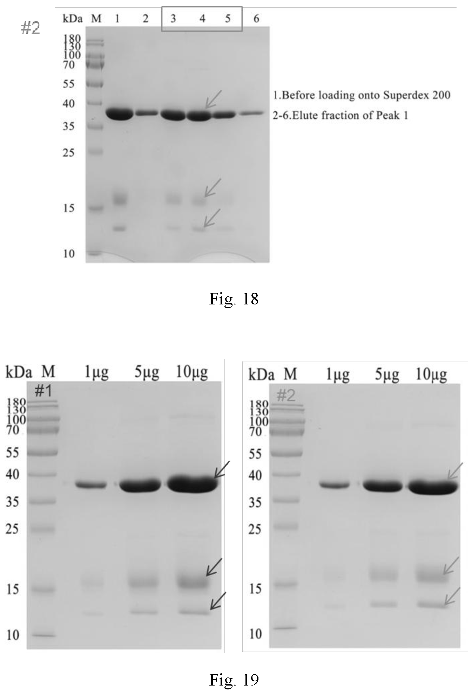

Ion exchange chromatography using a Q HP (5 mL) column is shown in FIG. 13, and the corresponding SDS-PAGE analysis is shown in FIG. 14. Further purification by SEC using a Superdex 200 10/300 column is shown in FIGS. 15-18. SDS-PAGE analysis of SEC-purified proteins is shown in FIG. 19.

Analytical size exclusion chromatography (aSEC) was performed under the following conditions:
Flow rate: 0.4 mL/min;
Buffer: PBS, pH 7.4;
Column: Superdex 200 Increase 5/150 GL.

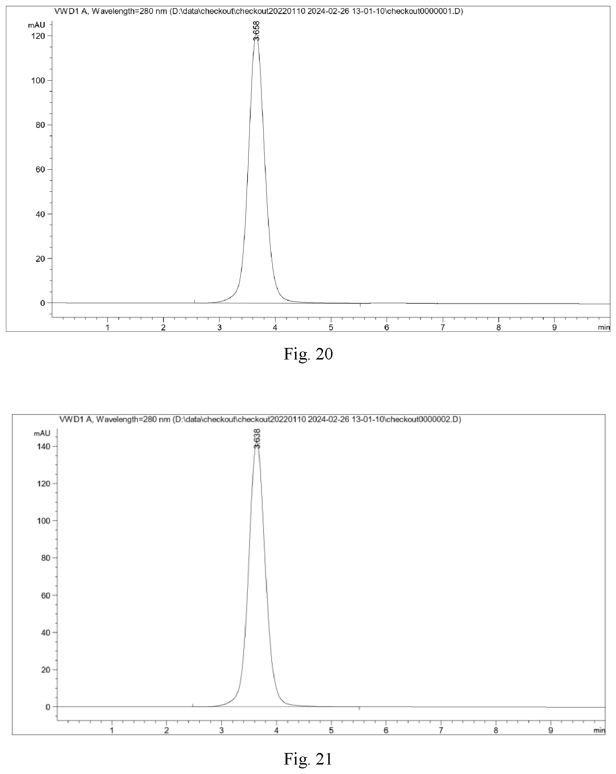

The aSEC results for Peak 1 are shown in FIG. 20, with a retention time of 3.658 min and a calculated apparent molecular mass (assuming a globular protein standard) of 122.4 kDa.

The aSEC results for Peak 2 are shown in FIG. 21, with a retention time of 3.638 min and a calculated apparent molecular mass of 126.7 kDa.

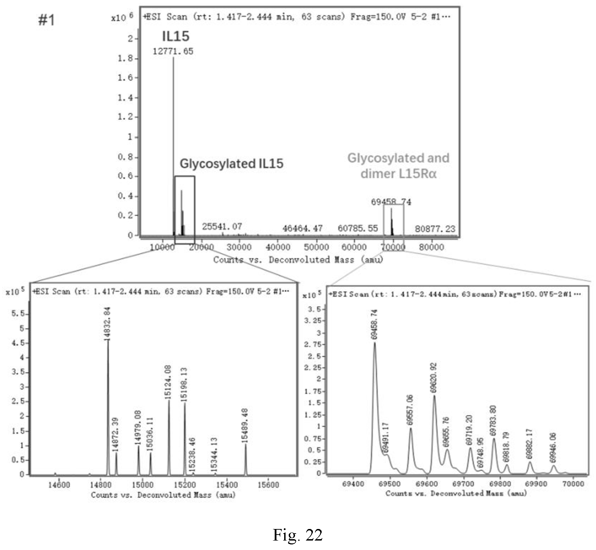

Native intact mass spectrometry analysis of Peak 1 is shown in FIG. 22. The major species detected include a non-glycosylated IL-15 polypeptide (12.772 kDa), glycosylated IL-15 polypeptides (14.833-15.489 kDa; major species at 14.833 kDa), and IL-15Rα-Fc fusion polypeptides (present as disulfide-linked homodimers) (69.459-69.946 kDa; major species at 69.459 kDa).

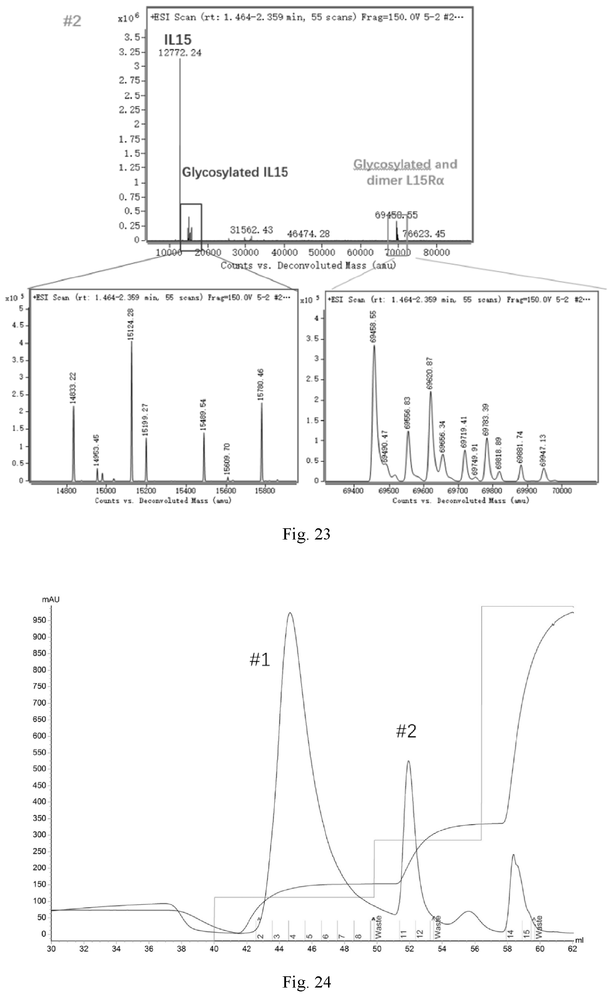

Native intact mass spectrometry analysis of Peak 2 is shown in FIG. 23. A similar distribution was observed, including a non-glycosylated IL-15 polypeptide (12.772 kDa), glycosylated IL-15 polypeptides (14.833-15.489 kDa; major species at 15.124 kDa), and IL-15Rα-Fc fusion polypeptides (69.459-69.946 kDa; major species at 69.459 kDa).

These results indicate that, in both Peak 1 and Peak 2 of QHJ-02V02, the IL-15 polypeptide is predominantly present in a non-glycosylated form.

### QHJ-02V03

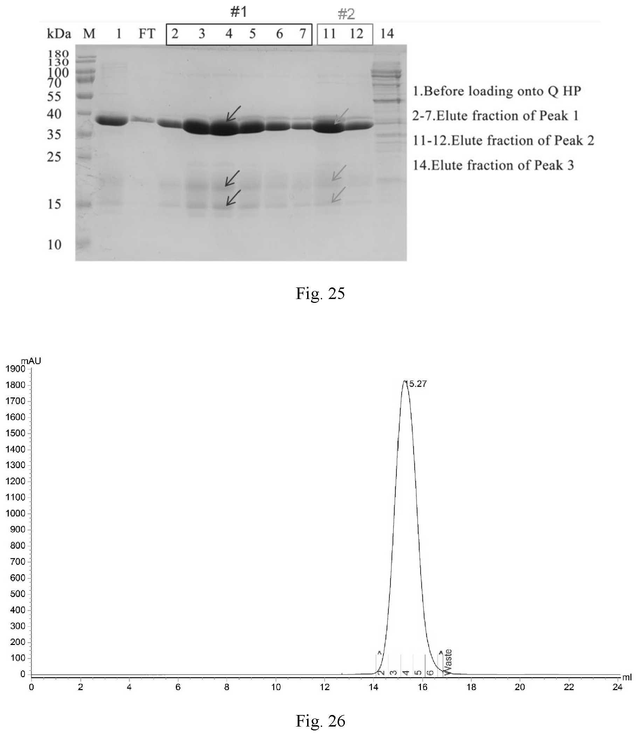

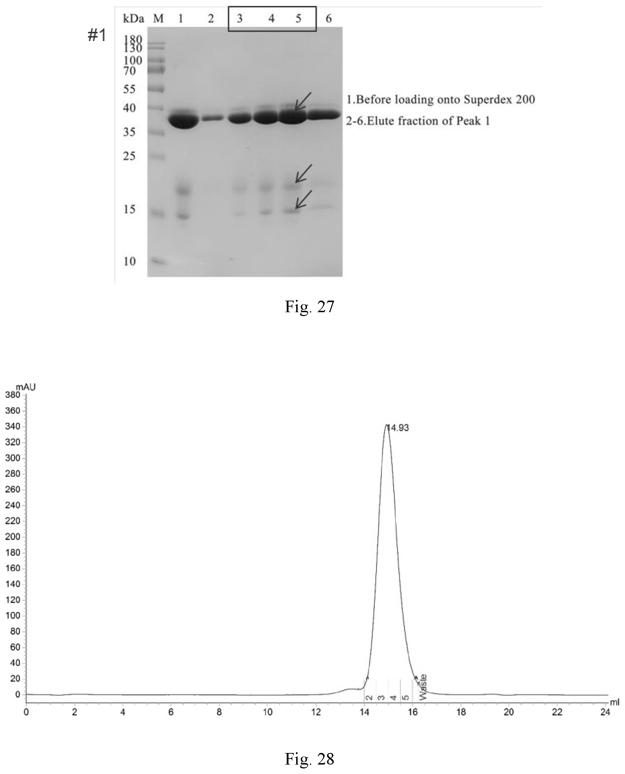

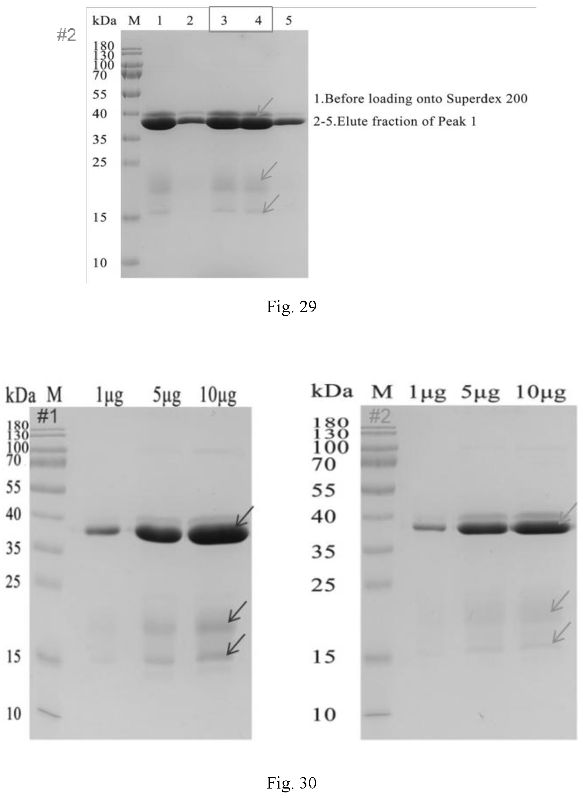

Ion exchange chromatography using a Q HP (5 mL) column is shown in FIG. 24, and the corresponding SDS-PAGE analysis is shown in FIG. 25. Further purification by SEC using a Superdex 200 10/300 column is shown in FIGS. 26-29. SDS-PAGE analysis of SEC-purified proteins is shown in FIG. 30.

Analytical size exclusion chromatography (aSEC) was performed under the same conditions as described above.

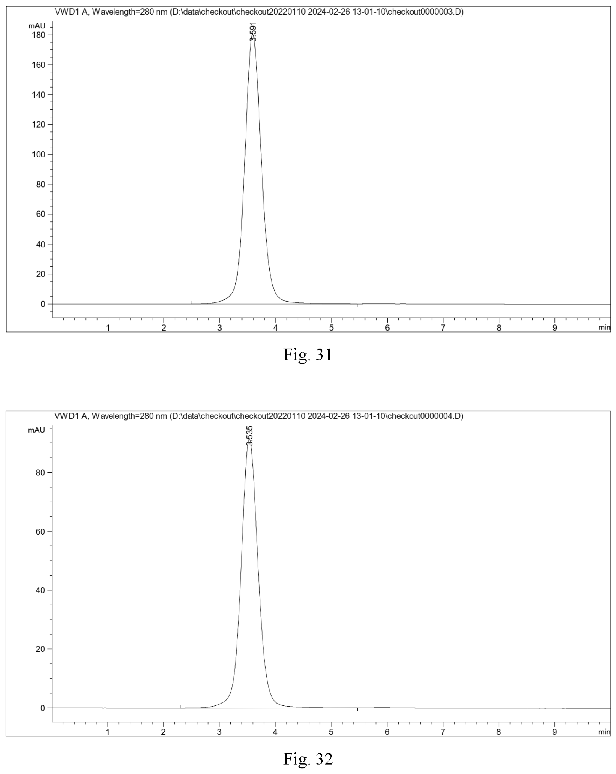

The aSEC results for Peak 1 are shown in FIG. 31, with a retention time of 3.591 min and a calculated apparent molecular mass of 137.3 kDa.

The aSEC results for Peak 2 are shown in FIG. 32, with a retention time of 3.535 min and a calculated apparent molecular mass of 151.0 kDa.

### Native intact mass spectrometry analysis

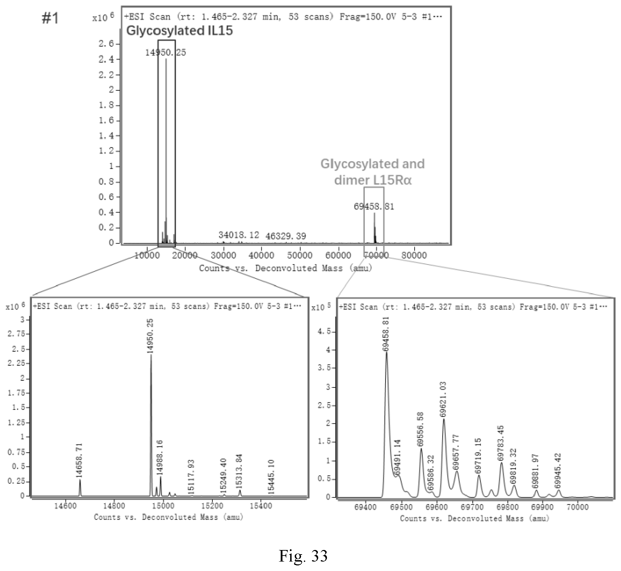

Native intact mass spectrometry analysis of Peak 1 is shown in FIG. 33. In contrast to QHJ-02V02, in which IL-15 is predominantly non-glycosylated, the IL-2/IL-15 chimeric polypeptide was predominantly present in a glycosylated form (14.95 kDa), and no non-glycosylated form was detected. The IL-15Rα-Fc fusion polypeptide exhibited a mass distribution consistent with that observed for QHJ-02V02 (69.459-69.946 kDa; major species at 69.459 kDa).

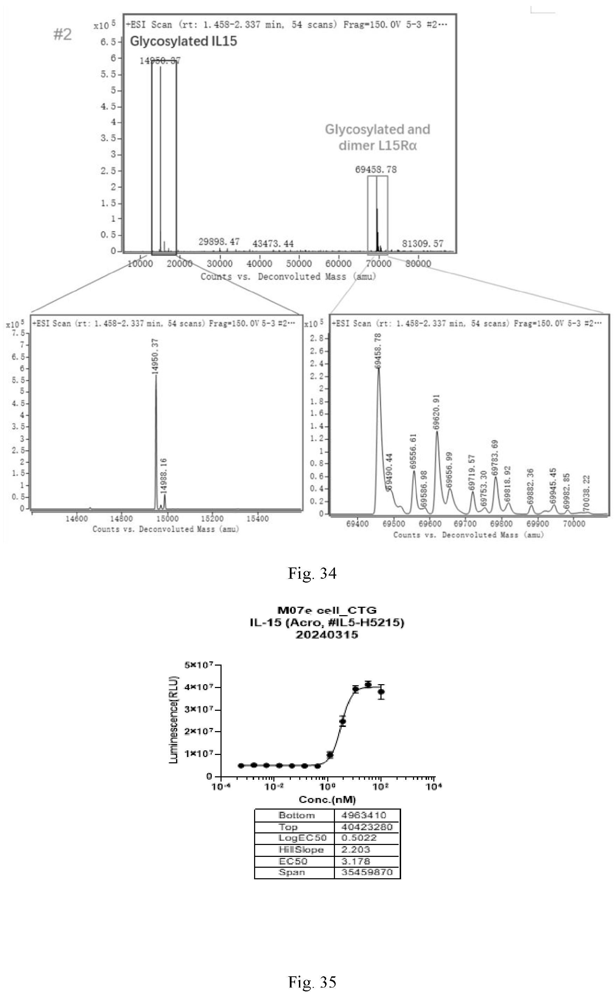

Native intact mass spectrometry analysis of Peak 2 is shown in FIG. 34. Similar results were observed, with the IL-2/IL-15 chimeric polypeptide predominantly present in a glycosylated form (14.95 kDa) and no detectable non-glycosylated species, while the IL-15Rα-Fc fusion polypeptide exhibited a consistent mass distribution.

In contrast to QHJ-02V02, the IL-2/IL-15 chimeric polypeptide is predominantly glycosylated, with no detectable non-glycosylated species under these conditions. The IL-15Rα-Fc component shows a consistent mass distribution.

This increased glycosylation is associated with improved in vivo stability, serum half-life, and biological activity, as supported by subsequent examples.

### Conclusion

SDS-PAGE, PNGase F digestion, SEC, and intact mass spectrometry demonstrates that incorporation of the IL-2-derived sequence into the IL-2/IL-15 chimeric polypeptide results in a substantially increased degree of glycosylation compared to IL-15 alone, including a predominance of glycosylated species and absence of detectable non-glycosylated forms. This reflects the introduction of additional glycosylation capacity by the IL-2 fragment and underlies the improved properties of the chimeric proteins.

### Example 5: Evaluation of the Stimulatory Effects of QHJ-02V02 and QHJ-02V03 on Mo7e Cell Proliferation

### Materials and Reagents

RPMI-1640 medium (Gibco, Cat. No. 22400089);
Fetal bovine serum (FBS) (Gibco, Cat. No. 10091130);
CellTiter-Glo^{®} Luminescent Cell Viability Assay kit (Promega, Cat. No. G7572);
96-well tissue culture-treated plates (Greiner, Cat. No. 655180);
Human IL-15 protein, premium grade (Acro Biosystems, Cat. No. IL5-H5215);
Mo7e cells.

### Methods

Protein concentration was determined using the Bradford method; proteins were stored in phosphate-buffered saline (PBS), pH 7.4.

### Experimental Procedures

Table 4 summarizes the positive control IL-15, test proteins, their theoretical molecular weights, and concentrations.

To assess cytokine-dependent cell proliferation, Mo7e cells were harvested during the logarithmic growth phase and washed twice with phosphate-buffered saline (PBS).

The cells were seeded into 96-well tissue culture plates at a density of 20,000 cells per 90 µL per well and incubated for 2 hours at 37°C in a humidified atmosphere containing 5% CO₂.

Following incubation, cells were treated with either the positive control IL-15 or the test proteins at a series of concentrations using a 12-point dilution curve starting at 100 nM with 3-fold serial dilutions. Treatments were performed in triplicate by adding 10 µL per well. IL-15 at the highest concentration was used as the positive control, and PBS was used as the negative control. The cells were then incubated for 72 hours under the same conditions.

After 72 hours of incubation, CellTiter-Glo^{®} reagent was added to each well. The plates were centrifuged at 1,000 rpm for 30 seconds and then incubated at room temperature with shaking for 15-30 minutes.

Luminescence was measured using an EnVision plate reader (PerkinElmer).

**Table 4. Test Proteins**

| No. | Protein | Theoretical MW* (kDa) | Concentration (mg/mL) | Concentration (mM) |
|---|---|---|---|---|
| 1 | IL-15 (Acro, #IL5-H5215) | 12.80 | 0.20 | 0.0156 |
| 2 | QHJ-02V02 (Peak 1) | 46.20 | 2.33 | 0.0504 |
| 3 | QHJ-02V02 (Peak 2) | 46.20 | 1.47 | 0.0318 |
| 4 | QHJ-02V03 (Peak 1) | 47.43 | 4.11 | 0.0866 |
| 5 | QHJ-02V03 (Peak 2) | 47.43 | 0.92 | 0.0194 |

| | | | | |
|---|---|---|---|---|
| *The theoretical molecular weight (kDa) refers to the molecular weight calculated based on the amino acid sequence of a single IL-15 molecule or a single IL-2/IL-15 chimeric polypeptide complexed with an IL-15Rα-Fc fusion polypeptide and does not correspond to the molecular weight of the final homodimeric complex. Glycosylation moieties are not included in the molecular weight calculation. | | | | |

### Data Analysis

Data were processed and analyzed using GraphPad Prism 8.0 software.

### Cell Proliferation Results

Results for the positive control IL-15 are shown in FIG. 35.

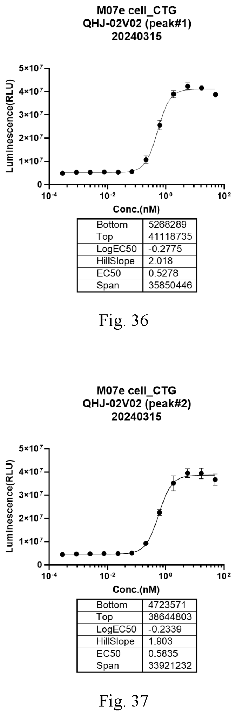

Results for QHJ-02V02 (Peak 1) are shown in FIG. 36.

Results for QHJ-02V02 (Peak 2) are shown in FIG. 37.

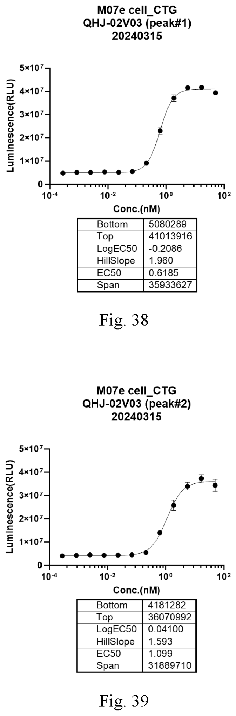

Results for QHJ-02V03 (Peak 1) are shown in FIG. 38.

Results for QHJ-02V03 (Peak 2) are shown in FIG. 39.

### Conclusions

Both QHJ-02V02 and QHJ-02V03 exhibited strong stimulatory effects on Mo7e cell proliferation. Compared with the positive control IL-15, both QHJ-02V02 and QHJ-02V03 increased Mo7e cell proliferation by approximately 3- to 6-fold based on EC50 values, with no statistically significant difference in EC50 observed between QHJ-02V02 and QHJ-02V03.

QHJ-02V02 Peak 1 and Peak 2 exhibited comparable proliferative activity, and QHJ-02V03 Peak 1 and Peak 2 also showed only minimal differences in activity.

### Example 6: In Vivo Evaluation of Tumor Inhibitory Effect of the reference protein QHJ-02V02 in B16F10 Melanoma Mouse Model

### Materials

The reference proteins QHJ-02V02 (Peak 1 and Peak 2), their stock concentrations and volumes are shown in Table 5.

**Table 5**

| No. | Protein | Code | Concentration (mg/mL) | Volume (ul) |
|---|---|---|---|---|
| 1 | QHJ-02V02(Peak 1) | QHJ-02V02p1 | 2.33 | 200 |
| 2 | QHJ-02V02(Peak 2) | QHJ-02V02p2 | 1.47 | 300 |

Both Peak 1 and Peak2 stock solutions have an appearance of colorless or almost colorless, clear isotonic liquid.

The positive control protein was human IL-15 obtained from ACRO Biosystems (Cat. No. IL5-H5215). It was expressed in HEK293 and provided as a lyophilized white powder.

### Experimental Animals

Six-week-old female C57BL/6N mice were obtained from Shanghai Shenchang Biotechnology Co., Ltd. The animals were housed in a specific pathogen-free (SPF) facility in individually ventilated cages (IVCs). Prior to the study, the animals were acclimatized for 3-7 days.

Room conditions were maintained at a temperature of 22 ± 2°C, relative humidity of 40-70%, and a 12 h/12 h light/dark cycle. The mice were provided with a standard sterilized laboratory diet and had ad libitum access to sterilized purified water.

### Instruments

Electronic balance from Shanghai Jingtian Electronic Instrument Co., Ltd. (Cat. No. JT201N); Disposable syringes from Shanghai Kindly Enterprise Development Group Co., Ltd. (Cat. No. K20190705).

### Procedures

### Dose Preparation

IL15 (2µg dose): 20µg dissolved in 2 mL PBS;
QHJ-02V02p1 (5µg dose): 50 µg QHJ-02V02p1 dissolved in 2 mL PBS;
QHJ-02V02p1 (15µg dose): 150 µg QHJ-02V02p1 dissolved in 2 mL PBS;
QHJ-02V02p2 (5µg dose): 50 µg QHJ-02V02p2 dissolved in 2 mL PBS;
QHJ-02V02p2 (15µg dose): 150 µg QHJ-02V02p2 dissolved in 2 mL PBS.
Administration volume was 200 µL for all.

### Experimental Animal Grouping and Evaluation Parameters

**Table 6**

| Group No. | N | Code | Dose* (µg) | Admin Route | Frequency |
|---|---|---|---|---|---|
| Group 1 | 8 | 1xPBS | 0 | | |
| Group 2 | 8 | IL15 | 2 | | |
| Group 3 | 8 | QHJ-02V02p1 | 5 | Intraperitoneal | 2 |
| Group 4 | 8 | QHJ-02V02p1 | 15 | | |
| Group 5 | 8 | QHJ-02V02p2 | 5 | | |
| Group 6 | 8 | QHJ-02V02p2 | 15 | | |

| | | | | | |
|---|---|---|---|---|---|
| The term "Dose" as used in the present invention refers to the amount administered to each animal per administration. | | | | | |

Each mouse was inoculated subcutaneously with 5 × 10⁶ B16F10 cells. On day 7 post-inoculation, tumor sizes were measured, and mice were randomly assigned into six groups. Two tumor-bearing mice with abnormal tumor development were excluded, resulting in 8 mice per group.

The dosing regimens were as follows: Group 1 (PBS, 200 µL, IP), Group 2 (IL-15, 2 µg, 200 µL, IP), Group 3 (QHJ-02V02p1, 5 µg, 200 µL, IP), Group 4 (QHJ-02V02p1, 15 µg, 200 µL, IP), Group 5 (QHJ-02V02p2, 5 µg, 200 µL, IP), and Group 6 (QHJ-02V02p2, 15 µg, 200 µL, IP). The theoretical molecular weight of IL-15 is 12.8 kDa; thus, the molar concentration corresponding to 2 µg/200 µL is 0.78 µM. The theoretical molecular weight of the QHJ-02V02 heterodimer (containing one IL-15 moiety) is 46.2 kDa; thus, the molar concentrations corresponding to 5 µg/200 µL and 15 µg/200 µL are 0.54 µM and 1.62 µM, respectively.

The day after grouping was designated as Day 1, and dosing was initiated on Day 2 (first administration). The second dose was administered on Day 6. Tumor volumes were measured over time.

### Measured Parameter

Tumor volume was calculated as: $V = 1 / 2 \times \left({L}_{long}\times {{L}_{short}}^{2}\right)$

### Data Analysis

Data was analyzed using Prism or SPSS software. P < 0.05 was considered statistically significant. Results are presented as Mean ± SD (standard deviation).

### Experimental Results

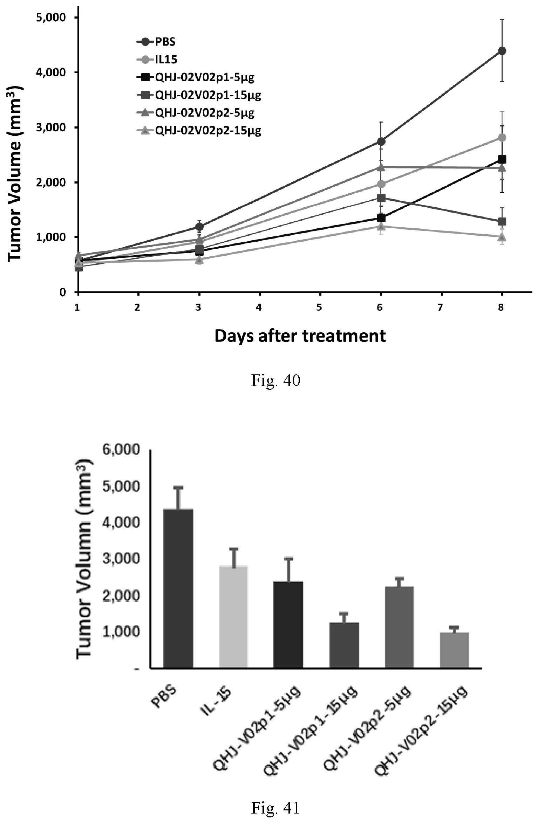

The change in tumor volume during the dosing period is shown in FIG. 40, and the tumor volume statistics are shown in FIG. 41.

The statistical values between each pair of groups are shown in Table 7.

**Table 7**

| | PBS | IL-15 | QHJ-V02p1-5µg | QHJ-V02p1-15µg | QHJ-V02p2-5µg | QHJ-V02p2-15µg |
|---|---|---|---|---|---|---|
| PBS | / | - | ** | *** | * | *** |
| IL-15 | 0.08 | / | - | * | - | *** |
| QHJ-V02p1-5µg | 0.007 | 0.20 | / | - | - | - |
| QHJ-V02p1-15µg | 0.001 | 0.049 | 0.57 | / | * | - |
| QHJ-V02p2-5µg | 0.045 | 0.81 | 0.23 | 0.04 | / | *** |
| QHJ-V02p2-15µg | 0.00005 | 0.00005 | 0.07 | 0.13 | 0.0004 | / |

The mean tumor volumes for Groups 1-6 were 4395.88, 2817.34, 2419.32, 1283.11, 2267.43, and 1007.59 mm³, respectively. Compared with the PBS control group (Group 1), all treatment groups (Groups 2-6) exhibited reduced tumor volumes. All test groups demonstrated improved efficacy relative to the positive control group (IL-15). The high-dose groups (Groups 4 and 6, 15 µg) showed greater tumor reduction than the low-dose groups (Groups 3 and 5, 5 µg). No statistically significant difference was observed between QHJ-02V02p1 and QHJ-02V02p2 at corresponding dose levels.

In summary, all treatment groups demonstrated antitumor activity in the subcutaneous melanoma mouse model. The high-dose groups (15 µg) exhibited greater efficacy than the low-dose groups (5 µg). QHJ-02V02p1 and QHJ-02V02p2 showed comparable efficacy.

These results indicate that the reference protein is effective in the treatment of subcutaneous melanoma.

### Example 7: In Vivo Evaluation of Inhibitory Effect on Tumor Metastasis in B16F10 Melanoma Lung Metastasis Model

### Objective of the Experiment

This experiment was conducted to evaluate the inhibitory effects of the test proteins in comparison with the reference protein on tumor metastasis in a B16F10 melanoma lung metastasis model.

### Tumor Cells

B16F10 melanoma cells were cultured in RPMI-1640 medium supplemented with 10% fetal bovine serum (FBS) at 37°C in a humidified atmosphere containing 5% CO₂. Cells were passaged every two days. Tumor cells in the logarithmic growth phase were harvested and used for in vivo implantation.

### Experimental Animals

Forty-five female C57BL/6N mice (7-8 weeks of age, 19-21 g) were obtained from Vital River Laboratory Animal Technology Co., Ltd. (Certificate No.: 20240409Abzz0619000169;
Experimental Animal Production License No.: SCXK (Zhejiang) 2024-0001). The Experimental Animal Use License (No.: SYXK (Shanghai)-2021-0021) is held by Lyvgen Biopharma (Shanghai) Co., Ltd.

Animals were housed in a specific pathogen-free (SPF) facility equipped with an individually ventilated cage (IVC) system under controlled environmental conditions (20-26°C, 40-70% relative humidity, 12 h light/12 h dark cycle). Mice were housed at 3-5 animals per cage, with ad libitum access to autoclaved food and water. Bedding was changed twice weekly.

All procedures involving animals were conducted in accordance with applicable AAALAC guidelines. Animals were monitored daily for general health, behavior, food and water intake, and clinical signs. Individual animals were identified by ear punching.

### Test Proteins

Table 8 summarizes the test proteins and their stock concentrations.

**Table 8**

| Proteins | Concentration (mg/mL) |
|---|---|
| QHJ02-V02 Peak1 | 2.33 |
| QHJ02-V02 Peak2 | 1.47 |
| QHJ02-V03 Peak1 | 4.11 |
| QHJ02-V03 Peak2 | 0.92 |

### Reagents and Instruments

The reagents and instruments used in this study are summarized in Table 9.

**Table 9**

| Item | Maker | Catalog No. | Batch No. |
|---|---|---|---|
| RPMI Medium 1640 | GIBCO | 11875-093 | 2717598 |
| FBS | GIBCO | 10099-141C | 2233792CP |
| Pen Strep | GIBCO | 15140-122 | 2585647 |
| 0.25% Trypsin-EDTA | GIBCO | 25200-072 | 2042303 |
| Trypan Blue | GIBCO | 15250-061 | 1309960 |
| PBS | Sigma | P5493 | SLBX1610 |
| 0.9% Saline | Double-Crane Pharm | NMPN H34023609 | 17071512Q |
| DMSO | SinoPharm | CAS NO67-78-5 | 20230402 |
| Anhydrous ethanol | SinoPharm | CAS 64-17-5 | 20221117 |
| Inverted Microscope | OLYMPUS | CKX31SF | - |
| CO₂ Incubator | Thermo | 371 | - |
| Centrifuge | Eppendorf | 5810R | - |
| Microbalance | Shanghai zanwei | ZA505AS | - |
| Balance | Acclulab | ALC-310.3 | - |
| ABSOLUTE Digimatic | Mitutoyo corp | 500-171-20 | - |

### Experimental Procedures

### Protein Solution Preparation for Animal Dosing

Protein dosing solutions were prepared from stock solutions as summarized in Table 11.

**Table 11**

| Group No. | Protein | Stock Concentration | Preparation Method | Dosing Solution Concentration (mg/mL) | Dose (µg) | Dose volume (mL) |
|---|---|---|---|---|---|---|
| 1 | PBS | / | Direct use | / | / | 0.2mL |
| 2 | IL-15 | 50µg (lyophilized) | 50 µg + 0.5 mL PBS (0.1 mg/mL stock); 150 µL → +1350 µL PBS | 0.01 | 2µg | 0.2mL |
| 3 | QHJ-02V02 | 2.33mg/mL | 20 µL stock ± 1844 µL PBS | 0.025 | 5µg | 0.2mL |
| 4 | | 1.47mg/mL | 100 µL stock ± 1860 µL PBS | 0.075 | 15µg | 0.2mL |
| 5 | QHJ-02V03 | 0.92mg/mL | 50 µL stock ± 1790 µL PBS | 0.025 | 5µg | 0.2mL |
| 6 | | 4.11 mg/mL | 33 µL stock ± 1775.4 µL PBS | 0.075 | 15µg | 0.2mL |

### Establishment of the Tumor Model

Each mouse was injected via the tail vein with 1 × 10⁵ B16F10 cells in 0.2 mL phosphate-buffered saline (PBS). A total of 45 mice (including 3 reserve animals) were inoculated. Dosing was initiated on the day of inoculation (Day 1). A total of three doses were administered on Days 1, 2, and 10. Dosing details are provided in Table 10.

**Table 10**

| Group No. | Number of Mice (N) | Test protein | Dose | Route | Frequency |
|---|---|---|---|---|---|
| 1 | 7 | PBS | / | IP | |
| 2 | 7 | IL-15 | 2µg | IP | One dose each on Days 1, 2, 10 |
| 3 | 7 | QHJ-02V02 | 5 µg | IP | |
| 4 | 7 | | 15 µg | IP | |
| 5 | 7 | QHJ-02V03 | 5 µg | IP | |
| 6 | 7 | | 15 µg | IP | |

Molar concentrations of test proteins are calculated same as in Example 6.

### Animal Observation

Animals were observed daily throughout the study. Clinical observations included behavior, food and water intake, body weight, coat condition, and any signs of morbidity or mortality.

### Evaluation Parameters

### Body Weight Monitoring

$BWL \left(%\right) = \left(BWi-{BW}_{0}\right) / {BW}_{0} \times 100 ,$ where BWi is the mean body weight at a given time point and BW₀ is the mean body weight on the first day of treatment.

### Body Temperature Monitoring

Body temperature was measured daily at a fixed time beginning one week after grouping. Particular attention was given to Days 10-16. Animals exhibiting a body temperature below 33°C were euthanized according to humane endpoint criteria.

### Lung Metastatic Lesion Counting

At study termination, lungs were excised, rinsed, and fixed in formalin. Visible metastatic nodules (black spots) on both anterior and posterior lung surfaces were counted.

### Endpoint of the Experiment

In this experiment, three doses were administered, and the experiment ended on Day 16. Animals were euthanized, lung tissue was completely excised, and tumor foci in the lungs were photographed and counted. Subsequently, the lung tissue was preserved in formalin.

### Data Analysis

Data are presented as mean ± standard deviation (SD). Statistical analysis was performed using SPSS 23.0 software. One-way ANOVA was used for comparisons, and P < 0.05 was considered statistically significant.

### Experimental Results

### Body Weight

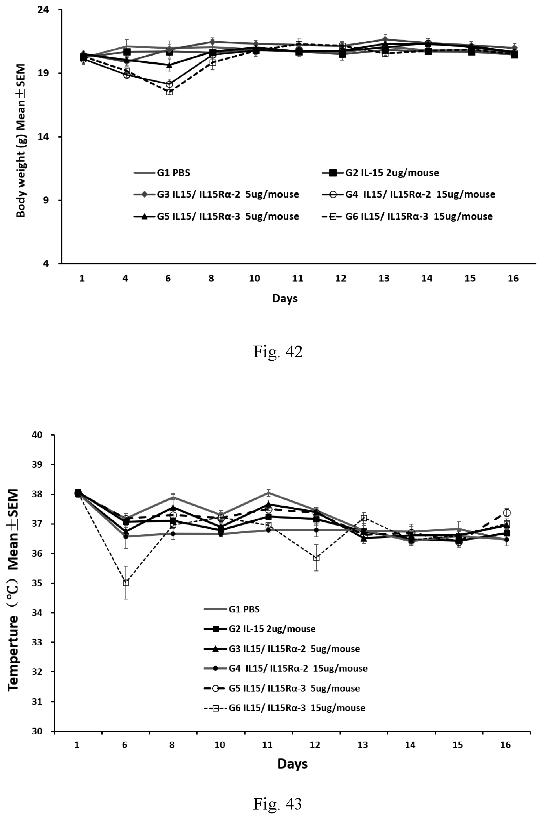

Body weight changes during the study are summarized in Table 12 and FIG. 42. No significant abnormal weight loss was observed in the treatment groups compared with the control group.

**Table 12: Mean Body Weight Change of Tumor-Bearing Mice During Dosing Period (Mean ± SD, n=7)**

| Day | G1 (PBS) | G2 (IL-15, 2µg) | G3 (QHJ-02V02, 5µg) | G4 (QHJ-02V02, 15µg) | G5 (QHJ-02V03, 5µg) | G6 (QHJ-02V03, 15µg) |
|---|---|---|---|---|---|---|
| D1 | 20.21±0.51 | 20.24±0.46 | 20.51±0.29 | 20.13±0.26 | 20.50±0.23 | 20.31±0.24 |
| D4 | 21.11±0.51 | 20.69±0.38 | 19.90±0.34 | 18.87±0.23 | 20.04±0.19 | 19.19±0.18 |
| D6 | 20.97±0.57 | 20.70±0.42 | 20.86±0.40 | 18.14±0.39 | 19.64±0.46 | 17.51±0.22 |
| D8 | 21.06±0.60 | 20.62±0.41 | 21.46±0.34 | 20.45±0.27 | 20.70±0.16 | 19.84±0.58 |
| D10 | 20.87±0.52 | 20.83±0.38 | 21.31±0.27 | 20.79±0.20 | 21.00±0.17 | 20.79±0.45 |
| D11 | 20.70±0.40 | 20.70±0.39 | 21.23±0.27 | 20.76±0.20 | 20.71±0.14 | 21.29±0.40 |
| D12 | 20.66±0.45 | 20.50±0.46 | 21.14±0.37 | 20.74±0.28 | 20.79±0.15 | 21.16±0.27 |
| D13 | 21.06±0.48 | 20.83±0.42 | 21.66±0.41 | 21.02±0.38 | 21.32±0.10 | 20.57±0.26 |
| D14 | 20.81±0.48 | 20.71±0.39 | 21.39±0.35 | 21.36±0.35 | 21.30±0.14 | 20.76±0.31 |
| D15 | 20.81±0.52 | 20.69±0.39 | 21.19±0.30 | 21.04±0.33 | 21.10±0.14 | 20.85±0.30 |
| D16 | 20.59±0.39 | 20.46±0.40 | 20.99±0.34 | 20.64±0.22 | 20.67±0.15 | 20.50±0.29 |

### Body Temperature

Body temperature data are summarized in Table 13 and FIG. 43. No unexpected temperature-related adverse effects were observed.

**Table 13: Mean Body Temperature Change of Tumor-Bearing Mice During Dosing Period (Mean ± SD, n=7)**

| Day | G1 (PBS) | G2 (IL-15, 2µg) | G3 (QHJ-02V02, 5µg) | G4 (QHJ-02V02, 15µg) | G5 (QHJ-02V03, 5µg) | G6 (QHJ-02V03, 15µg) |
|---|---|---|---|---|---|---|
| D1 | 38.03±0.14 | 38.07±0.05 | 38.01±0.10 | 38.03±0.08 | 38.04±0.08 | 38.03±0.13 |
| D6 | 37.17±0.18 | 37.07±0.13 | 36.74±0.15 | 36.57±0.41 | 37.17±0.18 | 35.01±0.56 |
| D8 | 37.90±0.08 | 37.11±0.18 | 37.56±0.45 | 36.67±0.20 | 37.30±0.19 | 36.97±0.12 |
| D10 | 37.30±0.15 | 36.79±0.15 | 36.90±0.06 | 36.66±0.07 | 37.20±0.11 | 37.21±0.19 |
| D11 | 38.04±0.11 | 37.24±0.11 | 37.66±0.15 | 36.79±0.09 | 37.50±0.04 | 36.96±0.06 |
| D12 | 37.46±0.10 | 37.16±0.19 | 37.40±0.11 | 36.79±0.21 | 37.37±0.12 | 35.86±0.45 |
| D13 | 36.77±0.24 | 36.74±0.20 | 36.51±0.17 | 36.79±0.15 | 36.63±0.18 | 37.20±0.18 |
| D14 | 36.74±0.17 | 36.47±0.21 | 36.61±0.13 | 36.41±0.13 | 36.70±0.29 | 36.46±0.12 |
| D15 | 36.83±0.25 | 36.43±0.15 | 36.61±0.17 | 36.59±0.08 | 36.40±0.19 | 36.60±0.15 |
| D16 | 36.46±0.20 | 36.69±0.13 | 36.96±0.13 | 36.47±0.13 | 37.39±0.13 | 37.01±0.15 |

### Lung Metastatic Lesions

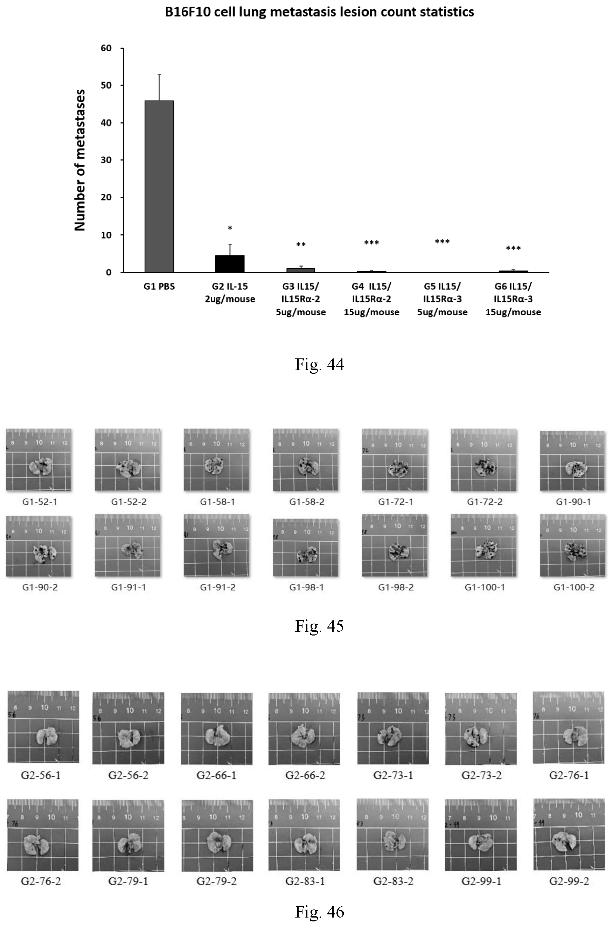

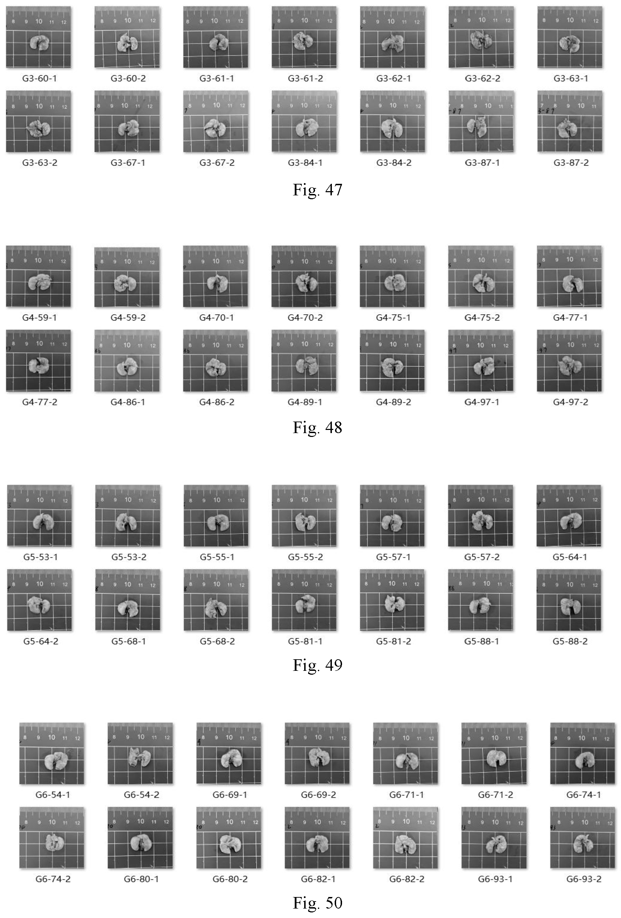

At the study endpoint (Day 16), lung metastasis data are summarized in Table 14 and FIGS. 44-50.

**Table 14: Inhibitory Effect of Test Proteins on the B16F10 Lung Metastasis Model (n=7)**

| Group | Numb er of Anima ls | Lung Metast atic Lesion s | Relative Tumor Burden Rate (%) | Metastas is Inhibitor y Rate * (%) | Metast asis-free Anima ls | Meta stasis -free Rate( %) | P Value¹ | P Value ² |
|---|---|---|---|---|---|---|---|---|
| G1 PBS | 7 | 45.9 | 100 | / | 0 | / | / | / |
| G2 IL-15 2 µg | 7 | 4.6 | 71.4 | 90.0 | 2 | 28.6 | 0.023 | / |
| G3 QHJ-02V02 5 µg | 7 | 1.1 | 42.9 | 97.5 | 4 | 57.1 | 0.001 | 0.346 |
| G4 QHJ-02V02 15 µg | 7 | 0.3 | 28.6 | 99.4 | 5 | 71.4 | <0.001 | 0.121 |
| G5 QHJ-02V03 5 µg | 7 | 0.00 | 0.00 | 100 | 7 | 100 | <0.001 | 0.025 |
| G6 QHJ-02V03 15 µg | 7 | 0.43 | 28.57 | 99.06 | 5 | 71.43 | <0.001 | 0.142 |

| | | | | | | | | |
|---|---|---|---|---|---|---|---|---|
| Note: $Metastasis inhibition rate \left(%\right) = \left(1-treated/control\right) \times 100$ P value¹: Statistical analysis between each treatment group (G2-G6) and the control group (G1). P value²: Statistical analysis between treatment groups G3-G6 and the positive control (G2) group. | | | | | | | | |

The mean number of lung metastatic lesions in the negative control group (Group 1) was 45.9, corresponding to a metastasis rate of 100%, confirming successful establishment of the model.

### Summary of the Experiment

This study evaluated the inhibitory effects of QHJ-02V02 and QHJ-02V03 on lung metastasis in a B16F10 melanoma lung metastasis model.

All treatment groups demonstrated significant inhibition of tumor metastasis compared with the control group. The metastasis rates in the QHJ-02V02 5 µg, QHJ-02V02 15 µg, QHJ-02V03 5 µg, and QHJ-02V03 15 µg groups were 42.86%, 28.57%, 0.00%, and 28.57%, respectively. The corresponding metastasis inhibition rates were 97.51%, 99.37%, 100.00%, and 99.06%, respectively.

No clear dose-dependent effect was observed between the two dose levels tested. Notably, QHJ-02V03 demonstrated superior anti-metastatic efficacy compared with QHJ-02V02, achieving complete inhibition of detectable lung metastasis at the 5 µg dose level.

These results indicate that QHJ-02V02 and QHJ-02V03 possess significant anti-metastatic activity, with QHJ-02V03 exhibiting enhanced efficacy, consistent with its improved biochemical properties, including higher expression levels and increased glycosylation.

### Example 8: Purification and Characterization of QHJ-02V13, QHJ-02V15, and QHJ-02M02

The general purification procedure for QHJ-02V13, QHJ-02V15, and QHJ-02M02 was as follows:
(1) Protein A resin was pre-equilibrated with 1× phosphate-buffered saline (PBS, pH 7.4) at 4°C. Subsequently, 100 mL of protein-containing supernatant (S fraction) was incubated with 0.5 mL of Protein A resin for 1 hour.
(2) The mixture was centrifuged at 1,000 rpm for 5 minutes, and the supernatant (flow-through fraction, FT) was collected. The resin was washed twice with 1 mL of PBS (pH 7.4).
(3) The bound protein was eluted three times with 1 mL of elution buffer (0.1 M glycine, pH 3.0).
(4) The eluates were immediately neutralized by adding 100 µL of neutralization buffer (1 M Tris, pH 8.0) per 1 mL of eluate.

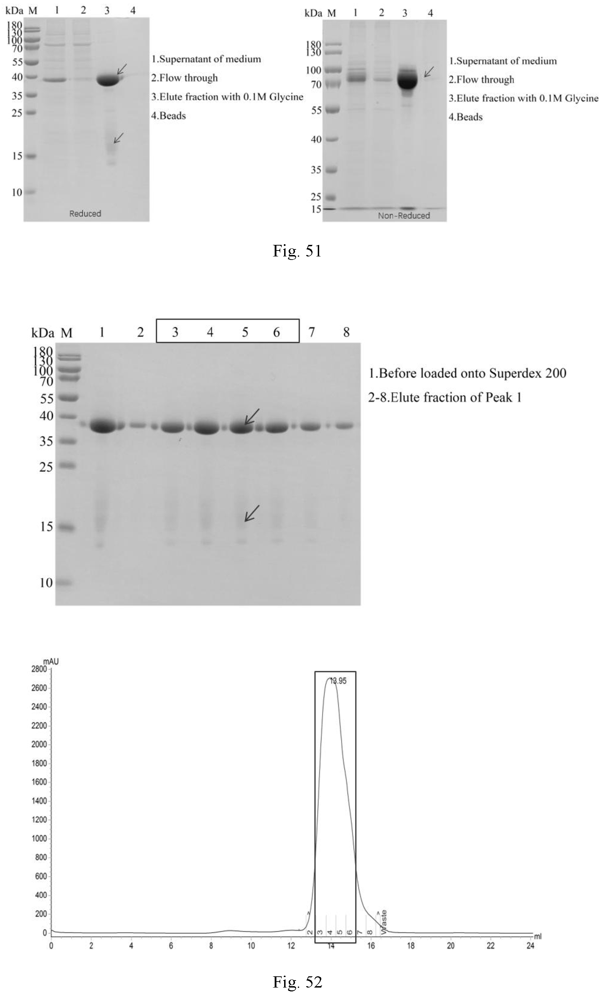

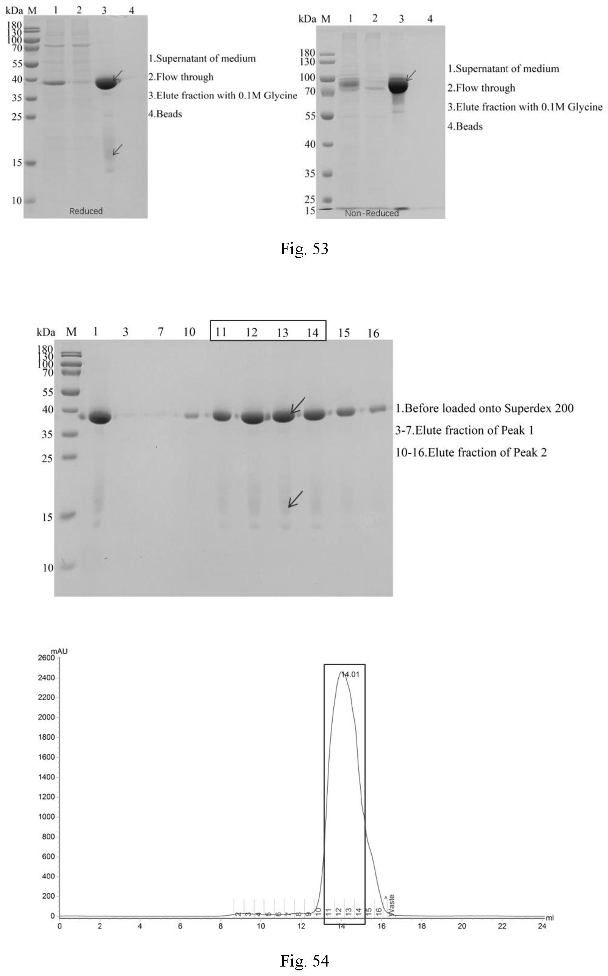

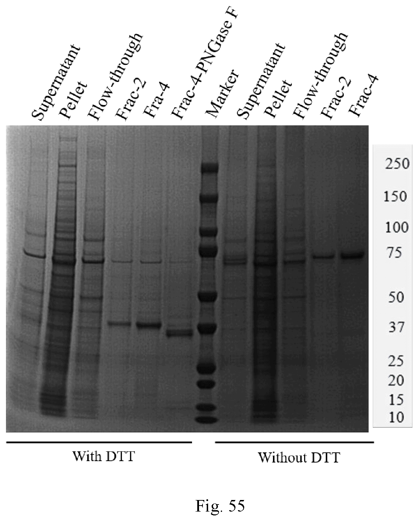

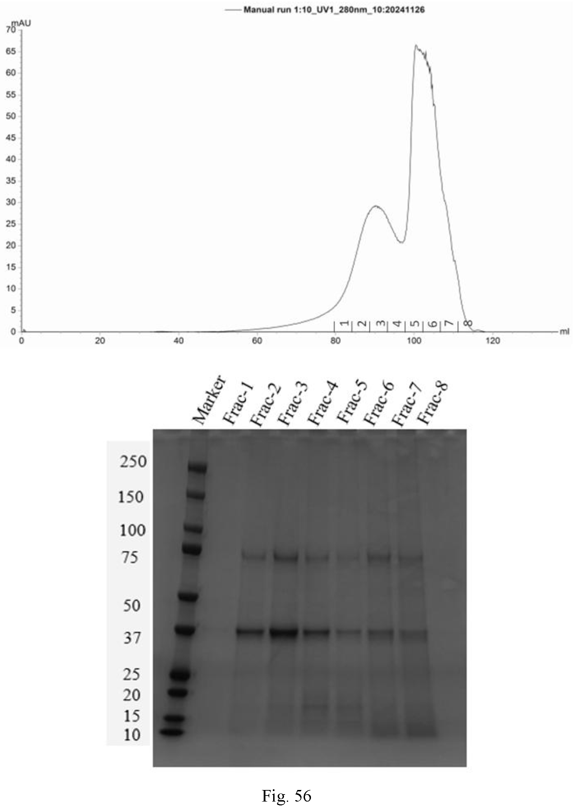

The Protein A eluate was concentrated and further purified by size exclusion chromatography (SEC) using a Superdex 200 10/300 column. The SEC running buffer was PBS (pH 7.4). SDS-PAGE analysis (12% gel) of QHJ-02V13, QHJ-02V15, and QHJ-02M02 following Protein A purification and subsequent SEC purification is shown in FIGS. 51-56.

### Example 9: Evaluation of the Stimulatory Effect of QHJ-02M02 on Mo7e Cell Proliferation

### Materials and Reagents

As described in Example 5.

### Methods

As described in Example 5.

### Experimental Procedures

Table 15 summarizes the positive control IL-15, test proteins, their theoretical molecular weights, and concentrations.

The experimental steps 1-5 are same as described in Example 5.

**Table 15**

| No. | Protein | Theoretical MW(kDa)* | Concentration (mg/mL) | Concentration(mM) |
|---|---|---|---|---|
| 1 | IL-15 (Acro, #IL5-H5215) | 12.80 | 0.20 | 0.0156 |
| 2 | QHJ-02M02-1 (Frac2-4) | 48.2 | 0.35 | 0.0072 |
| 3 | QHJ-02M02-2 (Frac5-7) | 48.2 | 0.37 | 0.0076 |

| | | | | |
|---|---|---|---|---|
| *The theoretical MW (kDa) is defined in Example 5 | | | | |

### Data Analysis

As described in Example 5.

### Results and Conclusion

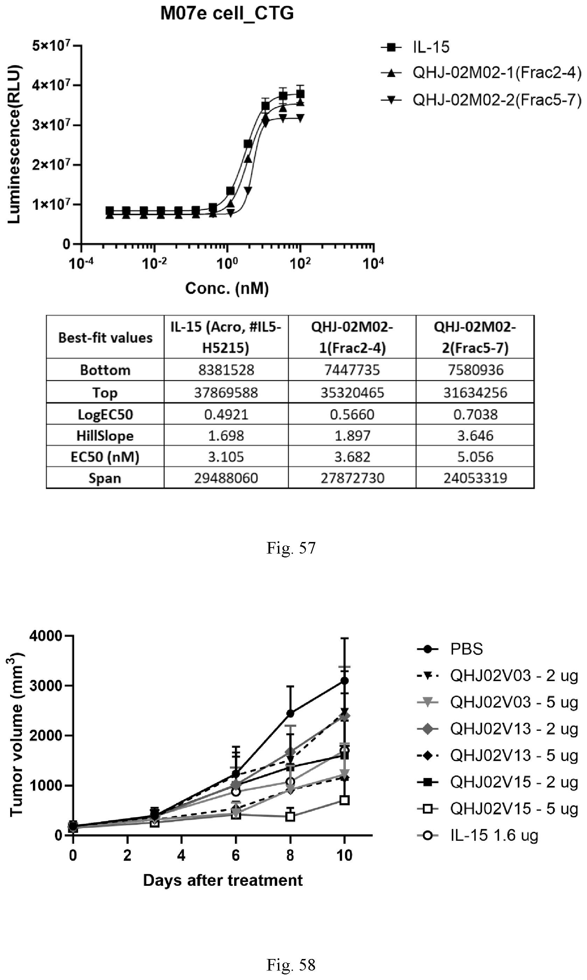

The results are shown in FIG. 57. QHJ-02M02 exhibited a stimulatory effect on Mo7e cell proliferation.

The EC₅₀ value of QHJ-02M02-1 (Frac 2-4, corresponding to SEC fractions 2-4) was comparable to that of IL-15. In contrast, QHJ-02M02-2 (Frac 5-7, corresponding to SEC fractions 5-7) exhibited weaker proliferative activity compared with QHJ-02M02-1.

These results indicate that QHJ-02M02 retains biological activity in stimulating Mo7e cell proliferation, with fraction-dependent differences in potency.

### Example 10: In Vivo Evaluation of Tumor Inhibition by QHJ-02V03, QHJ-02V13, and QHJ-02V15 in a B16F10 Melanoma Mouse Model

### Materials

Test proteins of QHJ-02V03, QHJ-02V13, and QHJ-02V15, their stock concentrations, and stock volumes are shown are shown in Table 16.

**Table 16**

| No. | Protein | Code | Concentration (mg/mL) | Volume(µL) |
|---|---|---|---|---|
| 1 | QHJ-02V03 | QHJ-02V03 | 4.11 | 100 |
| 2 | QHJ-02V13 | QHJ-02V13 | 6.24 | 200 |
| 3 | QHJ-02V15 | QHJ-02V15 | 8.23 | 200 |

| | | | | |
|---|---|---|---|---|
| *Appearance: Colorless or almost colorless, clear isotonic* liquid. | | | | |

The positive control IL-15 was from the same batch as described in Example 6.

### Experimental Animals, Instruments, and Housing Conditions

Unless otherwise specified, the experimental animals, instruments, and housing conditions were the same as those described in Example 6.

### Procedures

Unless otherwise specified, the experimental procedures were as described in Example 6.

### Dose Preparation

Test proteins (QHJ-02V03, QHJ-02V13, and QHJ-02V15) were diluted with 1× PBS to a final volume of 2 mL to achieve the required dosing concentrations (200 µL per mouse).

For each protein:
**2 µg dose:** 0.02 mg of protein was diluted to 2 mL with 1× PBS.
**5 µg dose:** 0.05 mg of protein was diluted to 2 mL with 1× PBS.

### Experimental Animal Grouping and Evaluation Parameters

Table 17 summarizes the grouping, dosing, and dosing frequency.

**Table 17**

| Group | Mouse N | Treatment | Dose(µg) | Route | Number of Administration |
|---|---|---|---|---|---|
| 1 | 7 | PBS | / | | |
| 2 | 7 | IL-15 | 1.6 | | |
| 3 | 7 | QHJ-02V03 | 2 | | |
| 4 | 7 | QHJ-02V03 | 5 | Intraperitoneal injection | 2 |
| 5 | 7 | QHJ-02V13 | 2 | | |
| 6 | 7 | QHJ-02V13 | 5 | | |
| 7 | 7 | QHJ-02V15 | 2 | | |
| 8 | 7 | QHJ-02V15 | 5 | | |

Each mouse was inoculated subcutaneously with B16F10 cells. Tumor measurement, grouping, dosing schedule (Day 2 and Day 6), and administration volume (200 µL, intraperitoneal) were the same as described in Example 6.

Theoretical molecular weights and corresponding molar concentrations were calculated as follows:
IL-15: 12.8 kDa → 1.6 µg/200 µL ≈ 0.63 µM
QHJ-02V03 and QHJ-02V13: 47.4 kDa
QHJ-02V15: 47.5 kDa
→ 2 µg ≈ 0.21 µM; 5 µg ≈ 0.53 µM

### Measured Parameter

Tumor volume calculation was performed as described in Example 6.

### Data Analysis

As described in Example 6

### Experimental Results

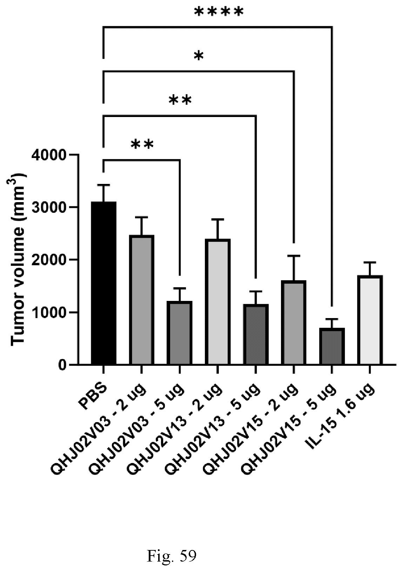

The change in tumor volume during the dosing period is shown in FIG. 58, and the tumor volume statistics are shown in FIG. 59.

The statistical values between each pair of groups are shown in Table 18.

**Table 18**

| | PBS | 02V03-2 µg | 02V03-5 µg | 02V13 -2 µg | 02V13 -5 µg | 02V15-2 µg | 02V15 -5 µg | IL-15-1.6 µg |
|---|---|---|---|---|---|---|---|---|
| PBS | / | - | ** | - | ** | * | **** | - |
| 02V03-2 µg | 0.860 | / | - | - | - | - | * | - |
| 02V03-5 µg | 0.002 | 0.134 | / | - | - | - | - | - |
| 02V13-2 µg | 0.744 | >0.9999 | 0.150 | / | - | - | * | - |
| 02V13-5 µg | 0.001 | 0.101 | >0.9999 | 0.113 | / | - | - | - |
| 02V15-2 µg | 0.027 | 0.556 | 0.986 | 0.614 | 0.970 | / | - | - |
| 02V15-5 µg | <0.0001 | 0.011 | 0.946 | 0.012 | 0.972 | 0.507 | / | - |
| IL-15-1.6 µg | 0.064 | 0.730 | 0.962 | 0.786 | 0.931 | >0.9999 | 0.424 | / |

At the endpoint of the experiment, the mean tumor volumes for the PBS group, QHJ-02V03-2 µg, QHJ-02V03-5 µg, QHJ-02V13-2 µg, QHJ-02V13-5 µg, QHJ-02V15-2 µg, QHJ-02V15-5 µg, and IL-15 groups were 3101.8, 2254.8, 1221.0, 2399.6, 1162.3, 1606.2, 705.9, and 1702.7 mm³, respectively.

Compared with the PBS group, all treatment groups exhibited reduced tumor volumes. In general, the 5 µg dose groups demonstrated greater tumor inhibition than the corresponding 2 µg dose groups. Among the tested proteins, the QHJ-02V15-5 µg group showed the greatest reduction in tumor volume, with the post-treatment tumor volume being approximately 22.76% of that observed in the PBS group.

The foregoing examples are provided for illustrative purposes only and are not intended to limit the present invention. Any modifications, equivalent substitutions, or improvements made within the scope of the invention as defined by the claims are intended to be included within the scope thereof.

## Claims

1. A protein comprising a heterodimeric unit formed by an IL-2/IL-15 chimeric polypeptide chain and an IL-15Rα-Fc fusion polypeptide chain,
wherein the IL-2/IL-15 chimeric polypeptide chain comprises an N-terminal IL-2 segment fused to a full-length IL-15 or a variant thereof, and
wherein the IL-15Rα-Fc fusion polypeptide chain comprises an IL-15Rα Sushi domain fused to an Fc region comprising CH2 and CH3 domains,
wherein two heterodimeric units are associated via Fc-mediated dimerization to form a homodimer of heterodimers;
wherein the protein is selected from QHJ-02V03, QHJ-02V05, QHJ-02V13, QHJ-02V15, and QHJ-02M02;
wherein:
QHJ-02V03 comprises
an IL-2/IL-15 chimeric polypeptide of SEQ ID NO: 7 or a polypeptide having at least 80% sequence identity thereto, and
an IL-15Rα-Fc fusion polypeptide of SEQ ID NO: 6 or a polypeptide having at least 80% sequence identity thereto;
QHJ-02V05 comprises
an IL-2/IL-15 chimeric polypeptide of SEQ ID NO: 8 or a polypeptide having at least 80% sequence identity thereto, and
an IL-15Rα-Fc fusion polypeptide of SEQ ID NO: 6 or a polypeptide having at least 80% sequence identity thereto;
QHJ-02V13 comprises
an IL-2/IL-15 chimeric polypeptide of SEQ ID NO: 9 or a polypeptide having at least 80% sequence identity thereto, and
an IL-15Rα-Fc fusion polypeptide of SEQ ID NO: 6 or a polypeptide having at least 80% sequence identity thereto;
QHJ-02V15 comprises
an IL-2/IL-15 chimeric polypeptide of SEQ ID NO: 9 or a polypeptide having at least 80% sequence identity thereto, and
an IL-15Rα-Fc fusion polypeptide of SEQ ID NO: 10 or a polypeptide having at least 80% sequence identity thereto;
QHJ-02M02 comprises
an IL-2/IL-15 chimeric polypeptide of SEQ ID NO: 9 or a polypeptide having at least 80% sequence identity thereto, and
an IL-15Rα-Fc fusion polypeptide of SEQ ID NO: 11 or a polypeptide having at least 80% sequence identity thereto.

2. A method for preparing the protein of claim 1, comprising:
constructing a first expression vector encoding the IL-2/IL-15 chimeric polypeptide chain and a second expression vector encoding the IL-15Rα-Fc fusion polypeptide chain;
co-transfecting host cells with the first expression vector, the second expression vector, and a transfection reagent; and
culturing the host cells, adding a transfection enhancer, continuing the culture, and
harvesting the protein.

3. A nucleic acid molecule system comprising a first nucleic acid molecule encoding an IL-2/IL-15 chimeric polypeptide chain and a second nucleic acid molecule encoding an IL-15Rα-Fc fusion polypeptide chain.

4. The nucleic acid molecule system according to claim 3, corresponding to the protein of claim 1, wherein:
when the protein is QHJ-02V03, the first nucleic acid molecule comprises SEQ ID NO: 12 and the second nucleic acid molecule comprises SEQ ID NO: 13, or sequences each having at least 70% sequence identity thereto;
when the protein is QHJ-02V05, the first nucleic acid molecule comprises SEQ ID NO: 14 and the second nucleic acid molecule comprises SEQ ID NO: 13, or sequences each having at least 70% sequence identity thereto;
when the protein is QHJ-02V13, the first nucleic acid molecule comprises SEQ ID NO: 15 and the second nucleic acid molecule comprises SEQ ID NO: 13, or sequences each having at least 70% sequence identity thereto;
when the protein is QHJ-02V15, the first nucleic acid molecule comprises SEQ ID NO: 15 and the second nucleic acid molecule comprises SEQ ID NO: 16, or sequences each having at least 70% sequence identity thereto; and
when the protein is QHJ-02M02, the first nucleic acid molecule comprises SEQ ID NO: 15 and the second nucleic acid molecule comprises SEQ ID NO: 17, or sequences each having at least 70% sequence identity thereto.

5. A system of expression vectors comprises a first vector comprising the first nucleic acid molecule according to claims 3 or 4 and a second vector comprising the second nucleic acid molecule according to claims 3 or 4.

6. A host cell comprising the system of nucleic acid molecules according to claim 3 or 4 or the system of expression vectors according to claim 5.

7. The protein according to claim 1, nucleic acid molecules according to claims 3 or 4, expression vectors according to claim 5, or the host cell according to claim 6, for use in the treatment of cancer.

8. The protein, nucleic acid molecules, expression vectors, or host cell for use according to claim 7, wherein the cancer comprises at least one cancer selected from melanoma (including metastatic melanoma, such as lung metastasis), acute granulocytic leukemia, chronic lymphocytic leukemia, acute lymphocytic leukemia, T-cell lymphoma, B-cell lymphoma, liver cancer, kidney cancer, small cell lung cancer, non-small cell lung cancer, nasopharyngeal carcinoma, colorectal cancer, pancreatic cancer, cervical cancer, ovarian cancer, breast cancer, bladder cancer, prostate cancer, acute myeloid leukemia, thyroid cancer, neuroblastoma, brain tumor, myeloma, and glioma.

9. The protein, nucleic acid molecules, expression vectors, or host cell for use according to claim 8, wherein the cancer is melanoma.

10. The protein, nucleic acid molecules, expression vectors, or host cell according to claim 9 for use in inhibiting metastasis of melanoma to the lung.

11. A pharmaceutical composition comprising as an active ingredient the protein according to claim 1, the nucleic acid molecules according to claim 3 or 4, the expression vectors according to claim 5, or the host cell according to claim 6.

12. The pharmaceutical composition according to claim 11, further comprising a pharmaceutically acceptable carrier.

13. The pharmaceutical composition according to claim 12, wherein the pharmaceutically acceptable carrier comprises one or more excipients, buffers, emulsifiers, stabilizers, diluents, binders, preservatives, lubricants, pH adjusters, cryoprotectants, and flavoring agents.

14. The pharmaceutical composition according to claim 11, wherein the composition is formulated for gastrointestinal or non-gastrointestinal administration.

15. The pharmaceutical composition according to claim 14, wherein the composition is formulated for non-gastrointestinal administration selected from injection, respiratory tract administration, dermal administration, mucosal administration, and body cavity administration.
